# EUROPEAN PATENT SPECIFICATION

(11) **EP 2 241 274 B1**
(45) Date of publication and mention of the grant of the patent: **01.02.2012**
(21) Application number: 10075066.0
(22) Date of filing: 07.12.2007
(51) Int. Cl.: A61B 17/32

(54) **Tissue removal devices**
Vorrichtungen zur Gewebeentfernung
Dispositifs de suppression des tissus

(30) Priority: 07.12.2006 US 869070 P
(43) Date of publication of application: 20.10.2010
(62) Divisional of application: 07865420.9
(73) Proprietor: Baxano, Inc., Mountain View, CA 94043 (US)
(72) Inventor: Schmitz, Gregory, Los Gatos CA 95032 (US); Bleich, Jeffery L., Palo Alto CA 94303 (US); Leguidleguid, Roy, Union City CA 94587 (US); Leguidleguid, Ronald, Fremont CA 94555 (US); Cantorna, Nestor C., Fremont CA 94538 (US); Wallace, Michael P., Pleasanton CA 94566 (US)
(74) Representative: Miller, James Lionel Woolverton

(56) References cited:
- WO-A1-01/08571
- US-A1- 2006 095 059
- US-A1- 2006 241 648
- US-A1- 2006 258 951

## Description

### BACKGROUND OF THE INVENTION

The present invention relates generally to medical/surgical devices . More specifically, the present invention relates to flexible tissue modification devices and methods.

A significant number of surgical procedures involve modifying tissue in a patient's body, such as by removing, cutting, shaving, abrading, shrinking, ablating or otherwise modifying tissue. Minimally invasive (or "less invasive") surgical procedures often involve modifying tissue through one or more small incisions or percutaneous access, and thus may be more technically challenging procedures. Some of the challenges of minimally invasive tissue modification procedures include working in a smaller operating field, working with smaller devices, and trying to operate with reduced or even no direct visualization of the tissue (or tissues) being modified. For example, using arthroscopic surgical techniques for repairing joints such as the knee or the shoulder, it may be quite challenging to modify certain tissues to achieve a desired result, due to the required small size of arthroscopic instruments, the confined surgical space of the joint, lack of direct visualization of the surgical space, and the like. It may be particularly challenging in some surgical procedures, for example, to cut or contour bone or ligamentous tissue with currently available minimally invasive tools and techniques. For example, trying to shave a thin slice of bone off a curved bony surface, using a small-diameter tool in a confined space with little or no ability to see the surface being cut, as may be required in some procedures, may be incredibly challenging or even impossible using currently available devices.

One area of surgery which would likely benefit from the development of less invasive techniques is the treatment of spinal stenosis. Spinal stenosis occurs when nerve tissue and/or the blood vessels supplying nerve tissue in the spine become impinged by one or more structures pressing against them, causing symptoms. The most common form of spinal stenosis occurs in the lower (or lumbar) spine and can cause severe pain, numbness and/or loss of function in the lower back and/or one or both lower limb.

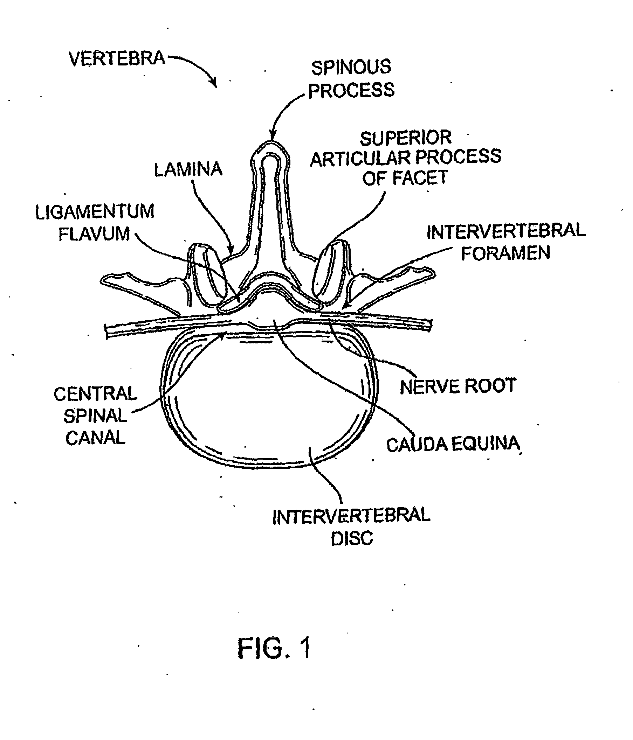

Fig. 1 is a top view of a vertebra with the cauda equina (the bundle of nerves that extends from the base of the spinal cord) shown in cross section and two nerve roots branching from the cauda equina to exit the central spinal canal and extend through intervertebral foramina on either side of the vertebra. Spinal stenosis can occur when the spinal cord, cauda equina and/or nerve root(s) are impinged by one or more tissues in the spine, such as buckled or thickened ligamentum flavum, hypertrophied facet joint (shown as superior articular processes in Fig. 1), osteophytes (or "bone spurs") on vertebrae, spondylolisthesis (sliding of one vertebra relative to an adjacent vertebra), facet joint synovial cysts, and/or collapse, bulging or herniation of an intervertebral disc. Impingement of neural and/or neurovascular tissue in the spine by one or more of these tissues may cause pain, numbness and/or loss of strength or mobility in one or both of a patient's lower limbs and/or of the patient's back.

In the United States, spinal stenosis occurs with an incidence of between 4% and 6% (or more) of adults aged 50 and older and is the most frequent reason cited for back surgery in patients aged 60 and older. Patients suffering from spinal stenosis are typically first treated with conservative approaches such as exercise therapy, analgesics, anti-inflammatory medications, and epidural steroid injections. When these conservative treatment options fail and symptoms are severe, as is frequently the case, surgery may be required to remove impinging tissue and decompress the impinged nerve tissue.

Lumbar spinal stenosis surgery involves first making an incision in the back and stripping muscles and supporting structures away from the spine to expose the posterior aspect of the vertebral column. Thickened ligamentum flavum is then exposed by complete or partial removal of the bony arch (lamina) covering the back of the spinal canal (laminectomy or laminotomy). In addition, the surgery often includes partial or complete facetectomy (removal of all or part of one or more facet joints), to remove impinging ligamentum flavum or bone tissue. Spinal stenosis surgery is performed under general anesthesia, and patients are usually admitted to the hospital for five to seven days after surgery, with full recovery from surgery requiring between six weeks and three months. Many patients need extended therapy at a rehabilitation facility to regain enough mobility to live independently.

Removal of vertebral bone, as occurs in laminectomy and facetectomy, often leaves the affected area of the spine very unstable, leading to a need for an additional highly invasive fusion procedure that puts extra demands on the patient's vertebrae and limits the patient's ability to move. Unfortunately, a surgical spine fusion results in a loss of ability to move the fused section of the back, diminishing the patient's range of motion and causing stress on the discs and facet joints of adjacent vertebral segments. Such stress on adjacent vertebrae often leads to further dysfunction of the spine, back pain, lower leg weakness or pain, and/or other symptoms. Furthermore, using current surgical techniques, gaining sufficient access to the spine to perform a laminectomy, facetectomy and spinal fusion requires dissecting through a wide incision on the back and typically causes extensive muscle damage, leading to significant postoperative pain and lengthy rehabilitation. Thus, while laminectomy, facetectomy, and spinal fusion frequently improve symptoms of neural and neurovascular impingement in the short term, these procedures are highly invasive, diminish spinal function, drastically disrupt normal anatomy, and increase long-term morbidity above levels seen in untreated patients.

US2006/0258951 concerns a method for locating neural tissue in a patient body which involves a tissue modifying member attached to a guidewire, and an actuator for activating the tissue modifying member to cut tissue. US 2006/0095059 concerns a flexible tissue-modification device which is reciprocated to remove tissue.

Therefore, it would be desirable to have less invasive methods and devices for modifying target tissue in a spine to help ameliorate or treat spinal stenosis, while inhibiting unwanted damage to non-target tissues. Ideally, such techniques and devices would reduce neural and/or neurovascular impingement without removing significant amounts of vertebral bone, joint, or other spinal support structures, thereby avoiding the need for spinal fusion and, ideally, reducing the long-term morbidity resulting from currently available surgical treatments. It may also be advantageous to have minimally invasive or less invasive tissue modification devices capable of treating target tissues in parts of the body other than the spine. At least some of these objectives will be met by the present invention.

The invention is defined in the independent claim. Preferred or optional features are set out in the dependent claims thereto.

### SUMMARY OF THE INVENTION

The devices of the present invention provide minimally invasive or less invasive modification of tissue in a patient. For the purposes of this application, the phrase "tissue modification" includes any type of tissue modification, such as but not limited to removing, cutting, shaving, abrading, shrinking, ablating, shredding, sanding, filing, contouring, carving, melting, heating, cooling, desiccating, expanding, moving, delivering medication or other substance(s) to tissue and/or delivering an implantable device (such as a stent) to tissue.

In accordance with the invention, there is provided a flexible tissue-modification device for removing target tissue from around a curved pathway within a patient by manually reciprocating both ends of the device while urging it against the target tissue, the device comprising:
a flexible elongate body having an axial length, a width and a thickness, wherein the axial length is greater than the width, and the width is greater than the thickness;
the body having two parallel sides formed of wire extending in the axial direction; and
characterised in that a plurality of cutting surfaces is connected to and extending between the two sides formed of wire across the width of the device.

Described herein are devices for modifying tissue in a patient that include: an elongate body having a proximal end and a distal end (wherein the elongate body comprises opposing first and second major surfaces laterally extending between the proximal and distal ends); a tissue collection region between the first and second surfaces; and one or more tissue modifying members disposed along the first major surface and configured to cut the tissue when the tissue modifying members are urged against the tissue.

The first and second major surfaces may be flexible. In some variations, the distal region comprises a flexible distal portion that includes the first and second major surfaces, and further comprising a proximal handle coupled with the proximal region of the elongate body.

These and other aspects and embodiments are described more fully below in the Detailed Description, with reference to the attached Drawings.

### BRIEF DESCRIPTION OF THE DRAWINGS

FIG. 1 is a top view of a vertebra with the cauda equina shown in cross section and two nerve roots branching from the cauda equina to exit the central spinal canal and extend through intervertebral foramina on either side of the vertebra.

FIG. 2A is a cross-sectional view of a patient's back and a side view of a flexible tissue modification device in position in a spine .

FIG. 2B is a diagrammatic view of a generic portion of a patient's body, showing target and non-target tissue, with the device of FIG. 2A in position to modify target tissue.

FIG. 2C is a side view of a tissue modification device .

FIG. 2D is a side view of a tissue modification device .

FIG. 3A is a view of a kit or system for modifying tissue .

FIG. 3B is a side view of a portion of the kit of FIG. 3B.

FIGS. 4A-4E demonstrate a method for inserting and using a flexible tissue modification device to modify tissue while inhibiting damage to non-target tissue,

FIG. 5A is a perspective view of a flexible portion of a tissue modification device.

FIGS. 5B and 5C are end-on and side views of blade and substrate portions of the portion of the device of FIG. 5A.

FIG. 6 is a perspective view of a portion of a flexible substrate and a wire saw tissue modifying member of a tissue modification device.

FIG. 7 is a perspective view of a portion of a flexible substrate and multiple wire saw tissue modifying members of a tissue modification device .

FIG. 8 is a perspective view of a portion of a flexible substrate and an abrasive surface tissue modifying member of a tissue modification device .

FIG. 9 is a perspective view of a portion of a flexible substrate and multiple tooth-like tissue modifying members of a tissue modification device .

FIG. 10 is a perspective view of a portion of a flexible substrate and a two-blade tissue modifying member of a tissue modification device .

FIG. 11 is a perspective view of a portion of a flexible substrate and multiple shark-tooth-shaped tissue modifying members of a tissue modification device .

FIG. 12 is a perspective view of a portion of a flexible substrate and multiple cheese-grater-shaped tissue modifying members of a tissue modification device .

FIG. 13 is a perspective view of a portion of a flexible substrate and multiple raised tissue modifying members of a tissue modification device .

FIG. 14 is a perspective view of a portion of a flexible substrate and multiple raised-flap tissue modifying members of a tissue modification device.

FIG. 15 is a perspective view of a portion of a flexible substrate and multiple rounded tissue modifying members of a tissue modification device .

FIG. 16 is a perspective view of a portion of a flexible substrate and multiple raised-flap tissue modifying members of a tissue modification device .

FIG. 17 is a perspective view of a portion of a flexible substrate and multiple, differently shaped tissue modifying members of a tissue modification device .

FIG. 18 is a perspective view of a portion of a flexible substrate and barbed-hook and raised-flap tissue modifying members of a tissue modification device .

FIG. 19 is a perspective view of a portion of a wire mesh flexible tissue modification device.

FIG. 20 is a perspective view of a portion of a flattened, hollow, flexible tissue modification device.

FIG. 21 is a perspective view of a portion of a flexible substrate and cheese-grater-shaped tissue modifying members coupled with a tissue capture member of a tissue modification device.

FIG. 22 is a perspective view of a portion of a moveable-link flexible tissue modification device.

FIG.23 is a perspective view of a portion of a flexible substrate and tissue modifying member of a tissue modification device .

FIG. 24 is a perspective view of a portion of a flexible substrate and tissue modifying member of a tissue modification device.

FIGS. 25A and 25B are perspective views of a portion of a flexible substrate and tissue modifying members of a tissue modification device .

FIGS. 26A-26C are side views of a portion of a flexible tissue device with a tissue capture member, demonstrating a method for tissue modification .

FIG. 27A is a side view demonstrating a method of making a portion of a flexible tissue modification device.

FIGS. 27B and 27C are side and perspective views, respectively, of a portion of a tissue modification device with a tissue capture member, such as may be constructed using a method as in FIG. 27A.

FIGS. 28A and 28B are side views of a portion of a flexible tissue modification device with a tissue capture member.

FIG. 28C is a side view of a portion of a flexible tissue modification device with a tissue capture member.

FIG. 29A is a perspective view of a flexible tissue modification device with a floating tissue capture member.

FIGS. 29B and 29C are end-on views of a flexible tissue modification device with a floating tissue capture member.

FIG. 30 is a side view of a portion of a hollow, flexible tissue modification device.

FIG. 31 is a side, cross-sectional view of a portion of a hollow, flexible tissue modification device with a tissue transport member .

FIG. 32 is a side, cross-sectional view of a portion of a hollow, flexible tissue modification device with a tissue transport member.

FIG. 33 is a side, cross-sectional view of a portion of a hollow, flexible tissue modification device with a tissue transport member.

FIG. 34A is a side, cross-sectional view of a portion of a hollow, flexible tissue modification device with a tissue transport member.

FIG. 34B is a side view of a proximal ratcheting mechanism portion of the hollow, flexible tissue modification device of FIG. 34A.

FIG. 34C is a top view of a portion of the substrate of the device of FIG. 34B.

FIG. 35 is a side, cross-sectional view of a portion of a hollow, flexible tissue modification device with a tissue transport member.

FIGS. 36A-36B show one variation of a tissue modification device having a tissue collection region.

FIG. 36C shows another variation of a tissue modification device having a tissue collection region.

FIGS. 37A and 37B show another variation of a tissue modification device having a tissue collection region.

FIGS. 38A and 38B show cross-sectional views of one variation of a removable and expandable tissue collection region.

FIGS. 39A and 39B show cross-sectional views of one variation of a removable and expandable tissue collection region, and FIG. 39C is a cross-section through just the removable surface forming part of the tissue collection region.

FIGS. 40A and 40B show cross-sectional views of one variation of a removable and expandable tissue collection region; and FIG. 40C is a side perspective view of a removable major surface that is expandable.

FIGS. 41A and 41B illustrate assembly of one variation of a tissue modification device including an expandable and removable tissue collection region.

FIG. 42 is a perspective view of another variation of a tissue modification device including a removable tissue collection region.

FIG. 43 is a perspective view of another variation of a tissue modification device including a removable tissue collection region.

FIG. 44A is a perspective view of a tissue modification device according to the invention including a tissue collection region; FIG. 44B is a section through the tissue modification device shown in FIG. 44A, and FIG. 44B is a perspective view of a cutting surface of the tissue modification device shown in FIG. 44A.

FIG. 45 is a side view of a tissue modification device in a position for performing a tissue modification procedure, showing a generic bone, soft tissue and non-target tissue, according to one embodiment of the present invention.

FIG. 46 is a side view of a tissue modification device with vertically oriented blades.

FIG. 47 is a perspective view of a flexible portion of a tissue modification device with vertically oriented blades.

FIG. 48 is a top view of a flexible portion of a tissue modification device with vertically oriented blades.

FIGS. 49A-49D are end-on views of flexible portions of various tissue modification devices with vertically oriented blades .

FIG. 50 is a top view of a flexible portion of a tissue modification device with vertically oriented blades .

FIGS. 51A-51E are end-on views of a flexible portion of a tissue modification device with vertically oriented blades, demonstrating a method for moving the device back and forth laterally in an intervertebral foramen .

FIG. 52 is a perspective view of a double-blade member for attachment to a flexible portion of a tissue modification device .

FIG. 53 is a perspective view of a double-blade member for attachment to a flexible portion of a tissue modification device .

FIG. 54 is a perspective view of a twelve-blade member for attachment to a flexible portion of a tissue modification device .

FIG. 55 is a perspective view of an eight-blade member for attachment to a flexible portion of a tissue modification device .

FIG. 56 is a side view of a flexible portion of a tissue modification device with vertically oriented blades.

FIG. 57 is a perspective view of a flexible portion of a tissue modification device with vertically oriented blades.

FIG. 58 is a top view of a flexible portion of a tissue modification device, demonstrating a method for forming vertically oriented blades .

FIGS. 59-76 are side views of various configurations of blades for use with tissue modification devices .

FIGS. 77-82 are cross-sectional views of various configurations of blades for use with tissue modification devices .

FIGS. 83A and 83B illustrate how tension in a flexible portion of a tissue modification device bent over a target tissue can urge blades or other tissue modification devices into the target tissue, and how torque to the rigid portion of the tissue modification device can maintain or alter an orientation of the flexible member and inhibit flipping of the flexible member.

FIG. 84A is a perspective view schematically illustrating shifting of the flexible member laterally along a target tissue by laterally translating the proximal rigid portion, and/or by pivoting the rigid portion about tissues along the site of insertion.

FIG. 84B schematically illustrates lateral translation and pivoting of the rigid portion to effect shifting of the flexible portion relative to the target tissue, and also schematically illustrates an optional rigid tubular shaft coupled to a distal handle to similarly allow shifting of the distal end of the flexible portion and provide enhanced control over target tissue remodeling and/or removal.

### DETAILED DESCRIPTION OF THE INVENTION

Various embodiments of tissue modification devices are provided. Although much of the following description and accompanying drawing figures generally focuses on surgical procedures in spine, in alternative embodiments, devices, systems and methods of the present invention may be used in any of a number of other anatomical locations in a patient's body. For example, in some embodiments, flexible tissue modification devices of the present invention may be used in minimally invasive procedures in the shoulder, elbow, wrist, hand, hip, knee, foot, ankle, other joints, or other anatomical locations in the body. Similarly, although some embodiments may be used to remove or otherwise modify ligamentum flavum and/or bone in a spine to treat spinal stenosis, in alternative embodiments, any of a number of other tissues may be modified to treat any of a number of other conditions. For example, in various embodiments, treated tissues may include but are not limited to ligament, tendon, bone, tumor, cyst, cartilage, scar, osteophyte, inflammatory tissue and the like. Non-target tissues may include neural tissue and/or neurovascular tissue in some embodiments or any of a number of other tissues and/or structures in other embodiments. In one alternative embodiment, for example, a flexible tissue modification device may be used to incise a transverse carpal ligament in a wrist while inhibiting damage to the median nerve, to perform a minimally invasive carpal tunnel release procedure. Thus, various embodiments described herein may be used to modify any of a number of different tissues, in any of a number of anatomical locations in the body, to treat any of a number of different conditions. The devices described with reference to figures 1-43 and 45-84B do not form part of the invention.

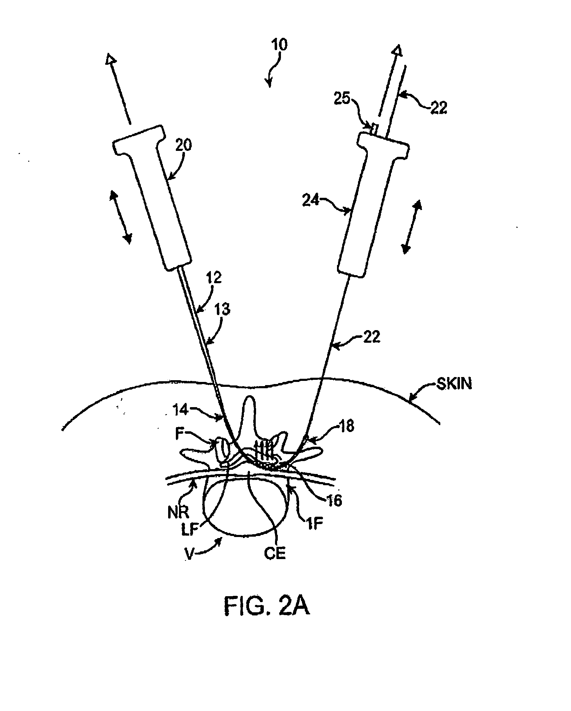

With reference now to Fig. 2A, a tissue modification device 10 according to one embodiment may suitably include a proximal handle 20 coupled with a shaft 12 having a proximal, rigid portion 13 and a distal, flexible portion 14 on which one or more tissue modifying members 16 may be disposed. A guidewire coupler 18 may be formed in (or attached to) flexible portion 14 at or near its distal end, for coupling with a guidewire 22, which in turn may be coupled with a guidewire handle 24 (or "distal handle"), which may include a tightening lever 25 for tightening handle 24 around guidewire 22.

Device 10 is shown percutaneously placed in position for performing a tissue modification procedure in a patient's spine, with various anatomical structures shown including a vertebra V, cauda equina CE, ligamentum flavum LF, nerve root NR, facet F, and intervertebral foramen IF. Various embodiments of device 10 may be used in the spine to remove ligamentum flavum LF, facet bone F, bony growths, or some combination thereof, to help decompress cauda equina CE and/or nerve root NR tissue and thus help treat spinal stenosis and/or neural or neurovascular impingement. Although this use of device 10 will not be continuously repeated for every embodiment below, any of the described embodiments may be used to remove ligamentum flavum alone, bone alone, or a combination of ligament and bone in the spine to treat neural impingement, neurovascular impingement and/or spinal stenosis.

In one embodiment of a method for modifying tissue using device 10, a distal end of guidewire 22 may be placed into the patient, along a curved path between target and non-target tissue, and out of the patient. A distal portion of guidewire 22 may then be coupled with guidewire handle 24, such as by passing guidewire 22 through a central bore in handle 24 and tightening handle 24 around guidewire 22 via tightening lever 25 or other tightening means. A proximal end of guidewire 22 may then be coupled with coupling member 18 and used to pull distal shaft portion 14 between target and non-target tissues. In some embodiments, device 10 may be advanced into the patient percutaneously, while in alternative embodiments, device 10 may be advanced through a small incision or larger incision. Once advanced into the patient, flexible distal shaft portion 14 may be advanced along a curved path between the target and non-target tissues, and in some instances may be pulled at least partway into an intervertebral foramen IF of the spine.

Proximal handle 20 and guidewire handle 24 may be pulled (or "tensioned"-solid/single-tipped arrows) to urge tissue modifying members 16 against the target tissue (in this case, ligamentum flavum LF). Generally, tissue modifying members 16 may be fixedly attached to (or formed in) one side or surface of distal portion 14, while an opposite side or portion of distal portion 14 faces non-target tissue, such as cauda equina CE and/or nerve root NR. The opposite side of distal portion 14 will generally be atraumatic and/or include an atraumatic cover, coating, shield, barrier, tissue capture member or the like. With tensioning force applied to device 10, handles 20, 24 may be used to reciprocate device 10 back and forth (solid/double-tipped arrows) to cause tissue modifying members 16 to cut, remove, shred or otherwise modify the target tissue. In various embodiments, for example, target tissue may include only ligamentum flavum LF, only bone, or a combination of both.

Reciprocation and tensioning may be continued until a desired amount of tissue is removed. Removed target tissue, in some embodiments, may be collected, captured or trapped between tissue modifying members 16 and/or in one or more tissue capture members or chambers (not shown). When a desired amount of target tissue has been removed, which may be determined, for example, by tactile feedback provided to the surgeon by device 10, by radiographic imaging, and/or by direct visualization (such as in an open surgical case), guidewire 22 may be released from distal handle 24, and device 10 may be removed from the patient's back. If desired, device 10 may be passed into the patient's spine again for additional tissue modification, and/or other devices may be passed into the spine.

Additional details of various methods for inserting and using device 10 are provided below.

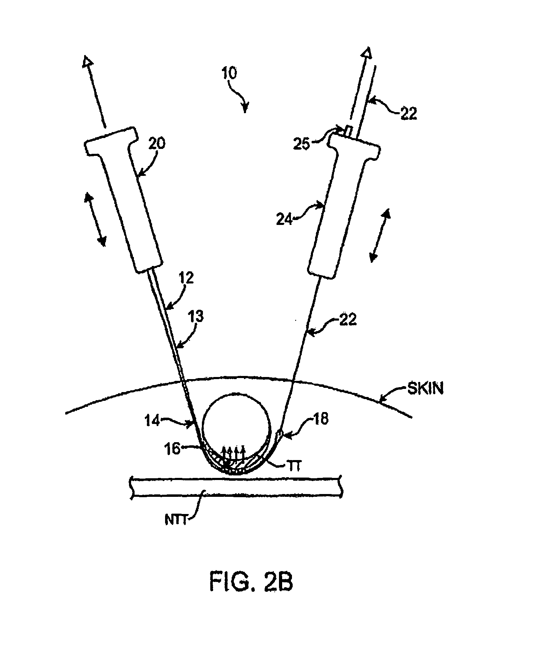

Referring now to Fig. 2B, in various embodiments, device 10 may be used in parts of the body other than spine to remove target tissue TT while avoiding harm to non-target tissue NTT. For example, target tissue TT may include soft tissue adhering to bone, such as ligament and/or cartilage, and/or may include bone. Non-target tissue NTT may include any nervous tissue, vascular tissue, an organ, or any other tissue that a surgeon may desire to leave unharmed by a surgical procedure. In one embodiment, for example, device 10 may be used to perform a minimally invasive carpal tunnel release procedure by releasing the transverse carpal ligament without damaging the median nerve. In some embodiments, such a procedure may be performed percutaneously with or without an endoscope. In other embodiments, device 10 may be used to remove cartilage and/or ligament from a knee or shoulder in a minimally invasive procedure. In yet another embodiment, device 10 may be used to perform a minimally invasive bunionectomy. Therefore, although the following discussion focuses primarily on various uses of alternative embodiments of device 10 in spine, any of a number of other anatomical structures may be operated upon in different embodiments.

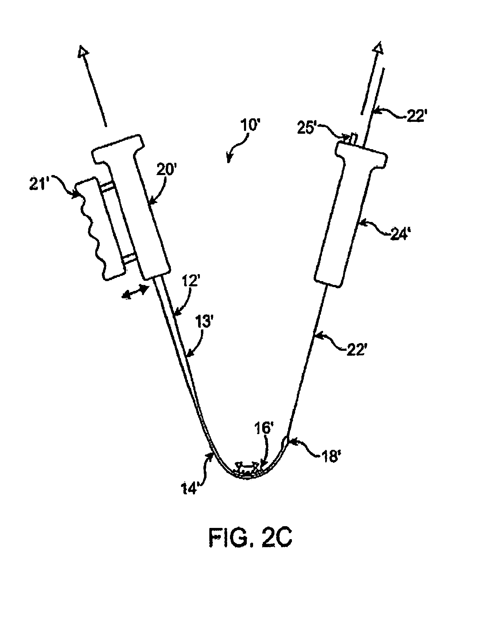

Referring now to Fig. 2C, in an alternative embodiment, a tissue modification device 10' may suitably include a proximal handle 20', including a squeeze actuator 21' and coupled with a shaft 12' having a proximal, rigid portion 13' and a distal, flexible portion 14'. One or more tissue modifying members 16' may be moveably coupled with one side of flexible portion 14', and a guidewire coupler 18' may be formed in (or attached to) flexible portion 14' at or near its distal end, for coupling with a guidewire 22' and thus a distal handle 24' with a tightening lever 25'.

In this alternative embodiment, squeeze actuator 21' may be coupled with moveable tissue modifying members 16' by any suitable means, such that actuating actuator 21' (double-headed, solid-tipped arrow) causes tissue modifying members 16' to reciprocate back and forth (double-headed, hollow-tipped arrow). In use, therefore, device 10' as a whole may be held relatively stationary, while tissue modifying members 16' are reciprocated. Proximal handle 20' and rigid proximal shaft portion 13' may be used to steer device 10' relative to target tissue, and of course device 10' may be moved in and out of the patient and/or the target tissue, but it may also be possible to hold device 10' relatively stationary while reciprocating tissue modifying members 16'. In various embodiments, squeeze actuator 21' may be replaced with any suitable mechanical actuator, such as a trigger, lever or the like.

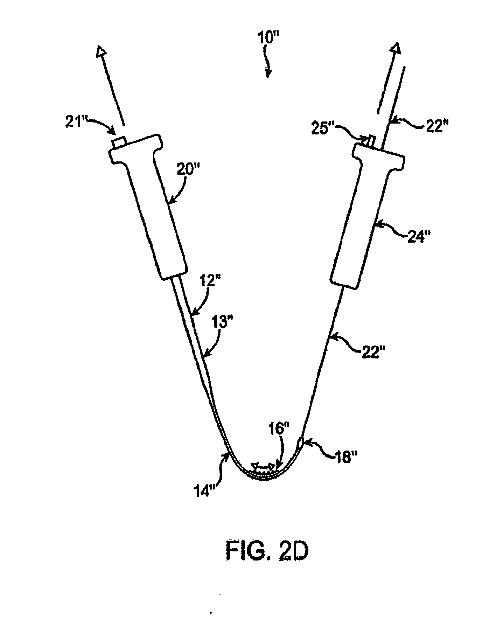

With reference now to Fig. 2D, in another alternative embodiment, a tissue modification device 10" may be similar to the previous embodiment but may include, instead of squeeze actuator 21', a button actuator 21" and a powered drive mechanism within handle 20". Pressing button actuator 21" may activate tissue modifying members 16" to reciprocate back and forth to modify tissue. In various alternative embodiments, button 21" may be replaced with any suitable actuator, such as a trigger, switch, dial or the like.

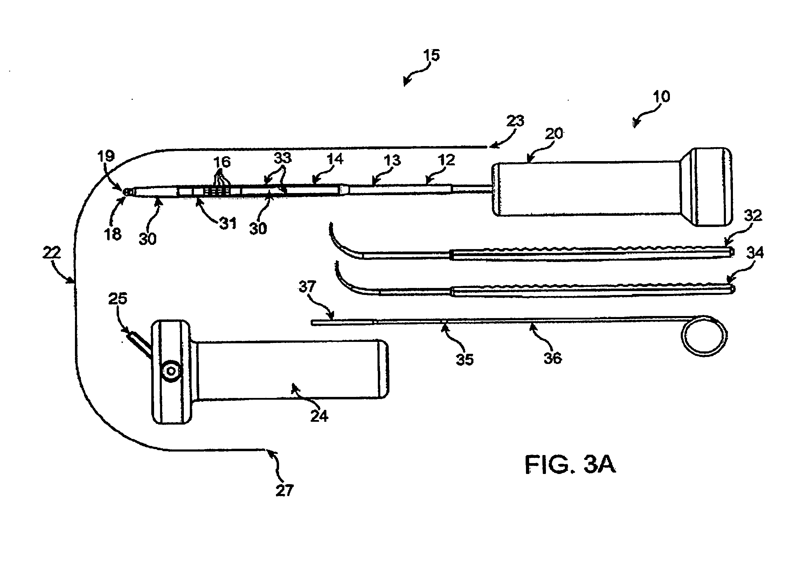

With reference now to Fig. 3A, in some embodiments tissue modification device 10 may be provided as a system (or "kit"), including the various components described above in reference to Figs. 2A and 2B. In some embodiments, a tissue modification system 15 or kit may suitably include device 10 of Figs. 2A and 2B, as well as one or more additional devices or components. For example, multiple guidewires 22 may be provided as part of system 15. In some embodiments, system 15 may also include one or more guidewire passage probes 32, 34 and a curved, flexible guide member 36. In one embodiment, for example, an ipsilateral access probe 32 and a contralateral access probe 34 may be provided. Curved guide member 36 is generally configured to pass through a lumen in each of probes 32, 34 and includes an inner lumen through which guidewire 22 may be passed. Guide member 36 may further include one or more depth marks 35 to indicate to a surgeon when guide member 36 has been passed a certain distance into probe 32, 34 and a stop 37 to limit passage of guide member 36 farther into probe 32, 34. In an alternative embodiment (not shown), such as might be used in a completely percutaneous procedure, probes 32, 34 may be replaced with an introducer needle, such as but not limited to a 14 gauge Touhy epidural needle or other size or type of epidural needle. In such an embodiment, guide member 36 may be designed to pass through the bore of the needle.

Guidewire 22 may be made of any suitable material, such as Nitinol or stainless steel, and may include a sharp distal tip 23, to facilitate passage of guidewire 22 through tissue, and a proximal shaped end 27 for coupling with guidewire coupler 18.

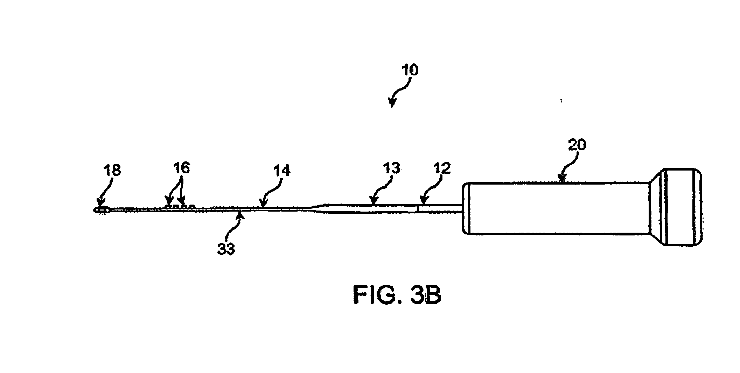

FIGS. 3A and 3B show proximal handle 20 and shaft 12 in greater detail than in previous figures. In the embodiment shown, four tissue modifying members 16 are fixedly attached to one side of flexible distal shaft portion 14, each comprising grooved blades with bi-directional cutting edges. In various alternative embodiments, any number of tissue modifying members 16 may be included, such as from one to twenty tissue modifying members 16. Furthermore, tissue modifying members 16 may have any of a number of different configurations, some of which are described below, such uni-directional blades, bi-directional blades, teeth, hooks, barbs, hooks, pieces of Gigli saw (or other wire saw), wires, meshes, woven material, knitted material, braided material, planes, graters, raised bumps, other abrasive surfaces, other abrasive materials, deliverable substances and/or the like.

In various embodiments, proximal shaft portion 13, distal shaft portion 14, tissue modifying members 16 and guidewire coupler 18 may be made of any suitable material (or materials), and may be made from one piece of material as a single extrusion or from separate pieces attached together. For example, in many embodiments, all of shaft 12 and guidewire coupler 18 may be made from one piece of material, and tissue modifying members 16 may be attached to distal shaft portion 14, such as by welding. In alternative embodiments, however, guidewire coupler 18 may be a separate piece attached to distal shaft portion 14 and/or tissue modifying members 16 may be formed in (rather than attached to) distal shaft portion 14. In yet another embodiment, distal shaft portion 14 may comprise a flat piece of material coupled with rigid proximal shaft portion 13, such as by welding. In some embodiments, shaft 12 may be formed from one piece of material, and distal shaft portion 14 may be flattened to derive its shape and flexibility. In some embodiments, one or more slits may be formed in distal shaft portion 14, to enhance its flexibility. In some embodiments, proximal shaft portion 13 may have a cylindrical shape. In some embodiments, proximal shaft portion 13, distal shaft portion 14, or both may be hollow. Alternatively, any portion of shaft 12 may be solid in some embodiments, such as to give proximal shaft portion 13 added rigidity.

In one embodiment, guidewire coupler 18 may include a slot 19, shaped to receive and hold guidewire proximal shaped end 27. In various embodiments, slot 19 may be located on the top surface of distal shaft portion 14, as shown, or on the bottom surface.
In some embodiments, an atraumatic cover 30 may be disposed over part of distal shaft portion 14, forming atraumatic edges 33 and an aperture 31 through which tissue modifying members 16 protrude. Cover 30 may be made of any suitable atraumatic material, such as any of a number of different polymers. In some embodiments, cover 30 may also serve to collect cut tissue. Cover 30 may be made of any suitable material, such as a polymer, examples of which are provided below. In some embodiments, cover 30 may be made from a porous or semi-permeable material and/or one or multiple holes may be formed in cover 30 to allow fluid to pass through cover 30, thus allowing a greater amount of solid material to be packed into a tissue collection portion of cover 30.

Fig. 3B is a side view of device 10. Tissue modifying members 16 may be seen extending above atraumatic edges 33 of cover 30 and having cutting edges facing both proximally and distally. In alternative embodiments, tissue modifying members 16 may have only uni-directional cutting edges, such as facing only proximally or only distally. In the embodiment shown, guidewire coupler 18 is formed as a loop at the distal end of distal shaft portion 14. Guidewire shaped end 27 may generally fit into slot 19 (not visible in Fig. 3B) to reside within the loop of guidewire coupler 18 during use. In other embodiments, guidewire coupler 18 may comprise a separate piece attached to the top side or bottom side of distal shaft portion 14.

The various components of device 10, including proximal handle 20, shaft 12, tissue modifying members 16, guidewire coupler 18, and cover 30, may be fabricated from any suitable material or combination of materials. Suitable materials include, for example, metals, polymers, ceramics, or composites thereof. Suitable metals may include, but are not limited to, stainless steel (303, 304, 316, 316L), nickel-titanium alloy, tungsten carbide alloy, or cobalt-chromium alloy, for example, Elgiloy® (Elgin Specialty Metals, Elgin, IL, USA), Conichrome® (Carpenter Technology, Reading, PA, USA), or Phynox® (Imphy SA, Paris, France). Suitable polymers include, but are not limited to, nylon, polyester, Dacron®, polyethylene, acetal, Delrin® (DuPont, Wilmington, DE ), polycarbonate, nylon, polyetheretherketone (PEEK), and polyetherketoneketone (PEKK). Ceramics may include, but are not limited to, aluminas, zirconias, and carbides. In some embodiments, one or more portions of shaft 12, for example, may be reinforced with carbon fiber, fiberglass or the like.

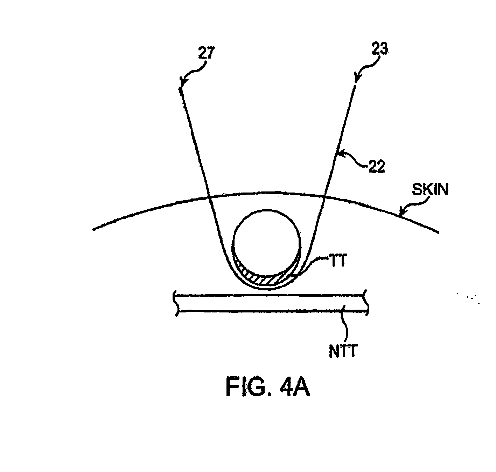

Referring now to Figs. 4A-4E, one embodiment of a method for modifying tissue using flexible tissue modification device 10 is demonstrated in greater detail. In these figures, a patient's skin, target tissue TT and non-target tissue NTT are shown diagrammatically, rather than as specific structures. In one embodiment, the method of Figs. 4A-4E may be employed in the spine, to remove ligamentum flavum, bone or both, with device 10 passing through an intervertebral foramen between two vertebrae, as shown in Fig. 2A. In other embodiments, other tissue in other areas of the body may be removed.

As shown in Fig. 4A, guidewire 22 with sharp tip 23 and shaped end 27 may be passed into the skin, between target and non-target tissue, and out of the skin. Guidewire 22 may be placed using a percutaneous method, such as with a needle, or using an open method, such as with a probe. In some embodiments, localization of neural tissue, such as with nerve stimulation on a guidewire passing probe or guidewire passing guide member may be used, to confirm that guidewire 22 is passed between target and non-target tissue.

In some embodiments where the method is performed in the spine, one or more substances or devices may be placed into the epidural space of the spine before or after placing guidewire 22, to create additional space between target tissues, such as ligamentum flavum, and non-target tissues, such as cauda equina and nerve root. Substances may include, for example, any of a number of fluids or gels, such as radiographic contrast medium. Devices may include, for example, a barrier or shield device. Injection of substances into the epidural space to create a safety zone is described in U.S. Patent Pub. No. 2006/0036211, titled "Spinal Ligament Modification Kit," assigned to X-Sten, Inc.

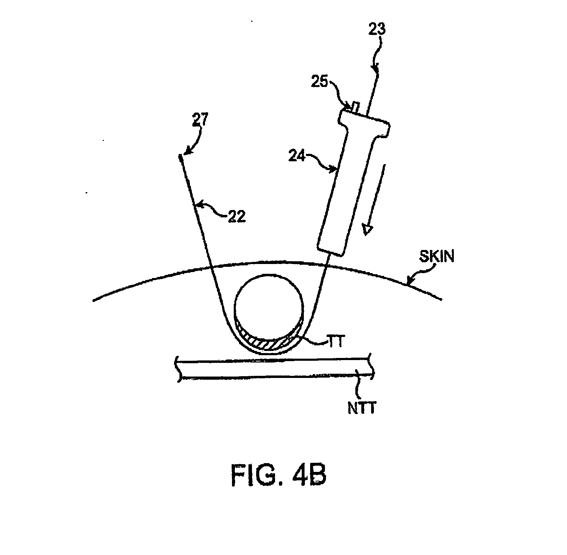

Referring to Fig. 4B, distal handle 24 may be passed over sharp tip 23 and tightened around guidewire 22, such as by moving tightening lever 25. Distal handle 24 may be coupled with guidewire 22 at this point in the process or at a later point, according to various embodiments.

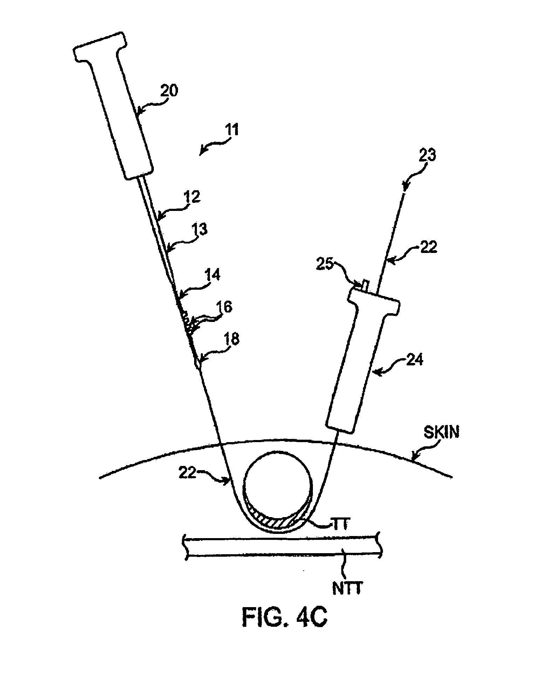

As shown in Fig. 4C, guidewire 22 may next be coupled with proximal device portion 11, by coupling shaped guidewire end 27 (not visible) with guidewire coupler 18. In the embodiment shown, for example, guidewire shaped end 27 may be placed into coupling member 18 (hollow-tipped arrow).

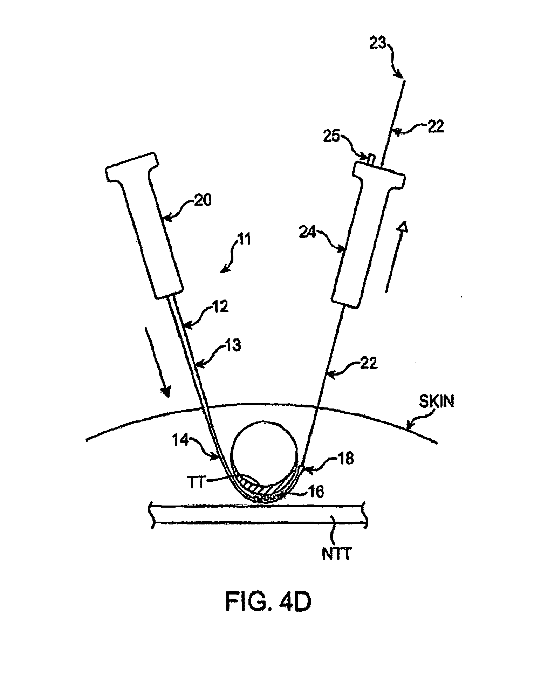

Referring to Fig. 4D, distal handle 24 may then be pulled (hollow-tipped arrow) to pull device 10 into the patient and to thus position tissue modifying members 16 in contact with target tissue TT. In some embodiments, such as when device 10 is used in a spinal procedure and passes through an intervertebral foramen, a surgeon or other physician user may use tactile feedback of device 10 passing into the foramen, such as when coupling member 18 and/or tissue modifying members 16 pass into the foramen, to determine when tissue modifying members 16 are positioned in a desired location relative to target tissue TT. Alternatively or additionally, a surgeon may confirm that a desired placement has been achieved by using radiographic imaging, such as fluoroscopy, direct visualization, such as in an open surgical case, or a combination of multiple methods.

In some embodiments in which device 10 is used in the spine to treat spinal stenosis and/or neural or neurovascular impingement, device 10 may be passed into the patient and to a position for modifying tissue without removing any vertebral bone. More specifically, in some embodiments, device 10 may be advanced into the patient, through an intervertebral foramen, and out of the patient without removing bone. This is contrary to the majority of current surgical methods for treating spinal stenosis, which typically include removal of at least some vertebral bone, such as performing a laminotomy or laminectomy, and which often remove significant amounts of vertebral lamina, spinous process, facet and/or pedicle bony tissue, simply to access the surgical site. In one embodiment, for example, device 10 may be advanced percutaneously into the patient, used to remove ligamentum flavum only, and withdrawn from the patient, without removing any vertebral bone.

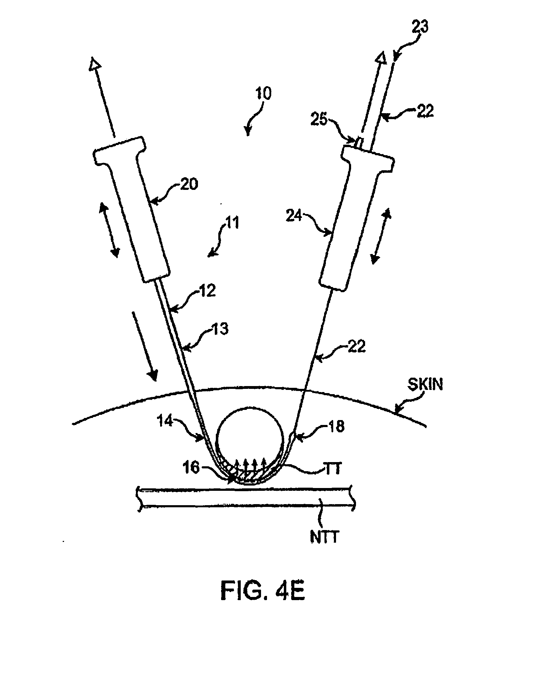

As shown in Fig. 4E, once tissue modifying members 16 are positioned as desired, relative to target tissue TT, proximal handle 20 and guidewire handle 24 may be pulled (hollow-tipped arrows) to urge tissue modifying members 16 against target tissue TT (solid-tipped, single-headed arrows). While maintaining pulling/tensioning force, handles 20, 24 may be used to reciprocate device 10 back and forth (solid-tipped, double-headed arrows) to remove target tissue TT. During a procedure, rigid proximal shaft portion 13 may be used to help steer device 10, or more specifically flexible distal shaft portion 14, relative to the target TT. For example, rigid shaft portion 13 may be used to move flexible portion 14 laterally or to pivot shaft 12 about an axis located along flexible portion 14. In one embodiment, for example, rigid portion 13 may be used to manipulate flexible portion 14 within an intervertebral foramen, such as by pivoting shaft 12 or moving flexible portion 14 laterally in a caudal and/or cephalad direction, relative to the patient. The rigidity of rigid proximal shaft portion 13 may generally facilitate such steering, as compared to a completely flexible device.

When a desired amount of tissue is removed, device 10 may be removed from the patient, such as by detaching guidewire handle 24 from guidewire 22 and pulling proximal handle 20 to withdraw device 10 and guidewire 22 out of the patient. In some embodiments, device 10 or an additional device may be reinserted into the patient and used in a second location to remove additional tissue. For example, in a spinal stenosis treatment procedure, device 10 may be used to remove tissue from (and thus decompress) a first intervertebral foramen and then may be removed and reinserted to remove tissue from a second foramen. This process may be repeated to remove tissue from any number of foramina. In one embodiment, device 10 may include a guidewire lumen, so that a guidewire may be placed into a second foramen while device 10 is in the epidural space of the patient. Device 10 may then be removed along with the first guidewire 22, attached to the second guidewire, and reinserted into the second foramen to remove tissue. In some embodiments, tissue may be removed from device 10 before reinserting device 10 into the patient to remove more tissue.

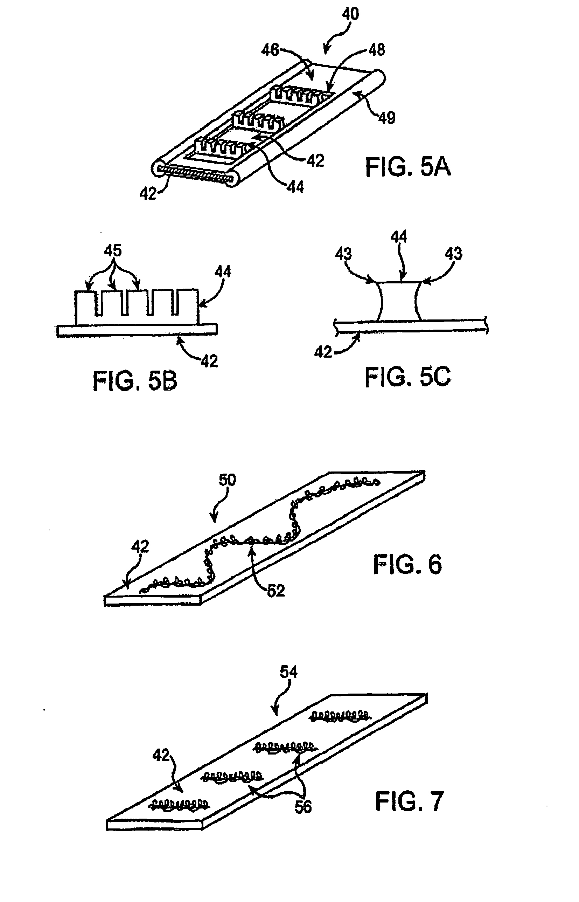

Referring now to Figs. 5A-5C, a flexible distal portion 40 of a flexible tissue modification device is shown, in various views. In Figs. 5A-5C and 6-25, various alternative embodiments of a flexible distal portion of a tissue modification device are shown in a generally straight configuration. However, all embodiments shown are flexible and thus may assume a curved configuration. The embodiments are shown in straight configuration for ease of illustration only.

In one embodiment, flexible distal portion 40 may include a substrate 42 (or "flexible, distal shaft portion"), multiple tissue modifying members 44 coupled with substrate 42, and an atraumatic cover 46 disposed over substrate 42 and forming an aperture 48 and atraumatic bumpers 49. Fig. 5B is an end-on view of substrate 42 and one of cutting members 44, which includes multiple teeth 45. Fig. 5C is a side view of substrate 42 and one of cutting members 44, showing that each cutting member 44 has two cutting edges 43 in this embodiment.

The embodiment of Fig. 5A includes three cutting members 44 comprising blades with multiple teeth 45 with grooves between them. Cutting members 44 in this and other embodiments may include any suitable material, such as but not limited to stainless steel or any of the materials listed previously above. Any number of cutting members 44 may be used, such as from one to twenty cutting members in various embodiments. Cutting members 44 may have any suitable height and may be spaced apart from one another at any suitable distances. In one embodiment, for example, cutting members 44 may have a height designed to protrude just slightly above the height of bumpers 49, so that cutting members 44 can cut tissue but do not protrude so high as to inhibit advancement or positioning of device in the patient. In some embodiments, cutting members 44 may be constructed as separate pieces and attached to substrate 42, such as by welding or gluing with adhesive. In some embodiments, cutting members 44 may be built by stacking layers of material to one another and attaching the stacks to form one piece. Cover 46 may be coupled with substrate using any known or later invented manufacturing technique, such as thermoforming, injection molding or the like.

In various alternative embodiments of distal portion 40 of Figs. 5A-5C, as well as in all embodiments described below and alternatives thereto, any number of cutting members 44 may be used, cutting members 44 may be made of any suitable material, and cutting members may be disposed along substrate 42 in any configuration, pattern or the like. Therefore, various alternative materials, numbers, patterns and the like of cutting members 44 will not be listed repeatedly for each alternative embodiment.

Referring now to Fig. 6, in another embodiment, a distal portion of a flexible tissue modification device 50 may include substrate 42 and a wire saw 52 coupled with substrate 42, such as by welding. In Fig. 6, as well as in subsequent Figs. 7-22, only a portion of each device embodiment including substrate 42 and one or more cutting members is shown, to simplify the drawing figures and description. Any of these embodiments may also include an atraumatic cover and/or other features, but for simplicity's sake, these features are not shown. Referring to the embodiment of Fig. 6, wire saw 52 may comprise any wire saw currently known or later invented, such as a Gigli saw, and may be attached to substrate 42 in any suitable pattern or configuration, such as in an S-shape pattern, as shown, or a zig-zag, straight-line or other pattern.

With reference to Fig. 7, in an alternative embodiment, a distal portion of a flexible tissue modification device 54 may include multiple pieces of wire saw 56 coupled with substrate 42. Again, these pieces of saw 56 may be attached in any pattern and by any means, such as by welding, and may comprise Gigli saw or other types of wire saw.

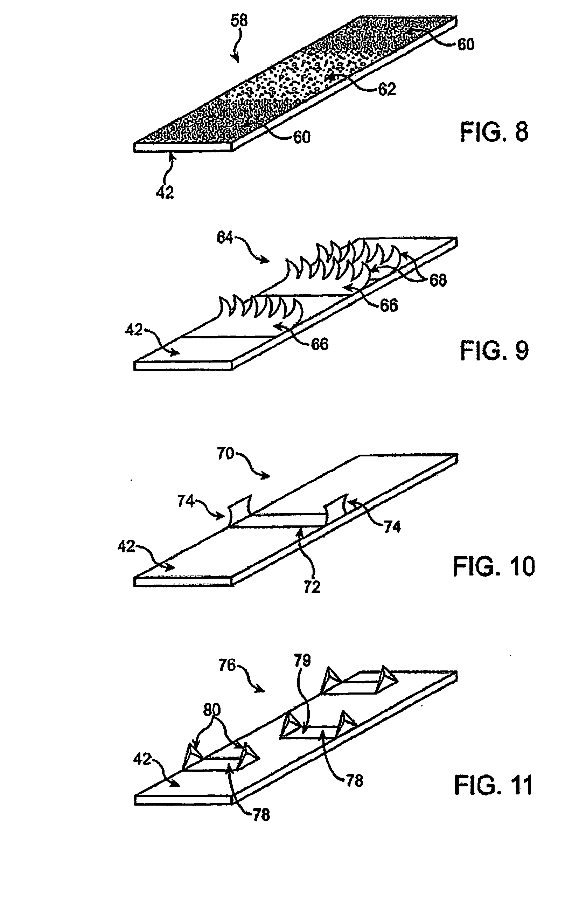

Fig. 8 shows a portion of another alternative embodiment of a flexible tissue modification device 58, in which abrasive materials 60, 62 are adhered to a surface of substrate. In some embodiments, only one type and/or grain of abrasive material 60 or 62 may be used, while other embodiments may include multiple types of material, multiple grains of material, or both. For example, in the embodiment shown, a finer grain of material 60 may be disposed at either end of a portion of coarser grain material 62. Such a variation in grains may provide varying degrees of tissue modification and/or the ability to remove greater amounts of tissue with a coarser grain 62 and provide a smoother finished surface to the tissue with the finer grain 60. In various embodiments, any abrasive materials 60, 62 may be used, and the materials may be adhered to substrate 42 via any method, such as adhering with adhesive or the like. One embodiment, for example, may include abrasive materials such as those described in U.S. Patent Pub. No. 2003/0225412, titled "Surgical Ribbon File . In another embodiment, substrate 42 may be treated in such a way as to have an abrasive surface, such as by sand blasting.

Referring to Fig. 9, in another alternative embodiment, a flexible tissue modification device 64 may include multiple tissue modifying members 66, each including multiple, curved teeth 68. Cutting members 66 may be made of stainless steel or other material(s). In some embodiments, cutting members 66 may be configured to primarily cut and/or shred ligamentous tissue, such as ligamentum flavum.

Referring to Fig. 10, in another alternative embodiment, a flexible tissue modification device 70 may include one or more tissue modifying members 72 coupled with a first major surface of a flexible substrate 42. Each tissue modifying member 72 may include a base 73 disposed between two blades 74, with a bend between base 73 and each blade 74. As will be described in greater detail below, each blade 74 may have a first end coupled with substrate 42 via base 73 and may extend to a second, cantilevered end. In some embodiments, each blade 74 may be substantially in-line (i.e., a side of blade 74 oriented at between about 0 degrees and about 45 degrees relative to a longitudinal axis of substrate 42) and may also be substantially vertical (i.e., a side of blade 74 forms an angle with the plane of substrate 42 of between about 45 degrees and about 90 degrees). Blades 74 may have any of a number of shapes, heights, lengths and the like, a number of embodiments of which will be described below. For example, Blades 74 may be designed, in one embodiment, specifically for cutting or slicing ligamentous tissue, such as ligamentum flavum.

Referring to Fig. 11, in another alternative embodiment, a flexible tissue modification device 76 may include multiple, laterally offset tissue modifying members 78 disposed laterally across a first major surface of a substrate flexible portion 42. In one embodiment, for example, each tissue modifying member 78 may include a base 79 with two substantially vertical blades 80 disposed at its opposite ends. Any suitable number of tissue modifying members 78 may be used in a given embodiment, such as but not limited to between two members 78 (four blades 78) and eight members 78 (16 blades 78), in alternative embodiments. Blades 80 may each have a triangular or "shark-tooth" shape, with two sharp cutting edges and a pointed cantilevered tip. In alternative embodiments, any of a number of other blade configurations may be used, some of which are described in greater detail below. In one embodiment, blades 80 may be designed specifically for cutting or slicing ligamentous tissue, such as ligamentum flavum. Alternatively, or additionally, blades 80 may be configured to cut bone. In one embodiment, each blade 80 may have a height approximately equal to or greater than a thickness of a ligamentum flavum. Such a blade 80 may be positioned in the spine to extend through ligamentum flavum and contact bone. When reciprocated, such a blade 80 may cut ligamentum flavum alone or may cut ligamentum flavum tissue and then, when it is removed, may also cut bone. Such a blade height and configuration may facilitate lateral steering of device 76. Various alternative embodiments of tissue modification devices having vertically oriented blades are described in greater detail below.

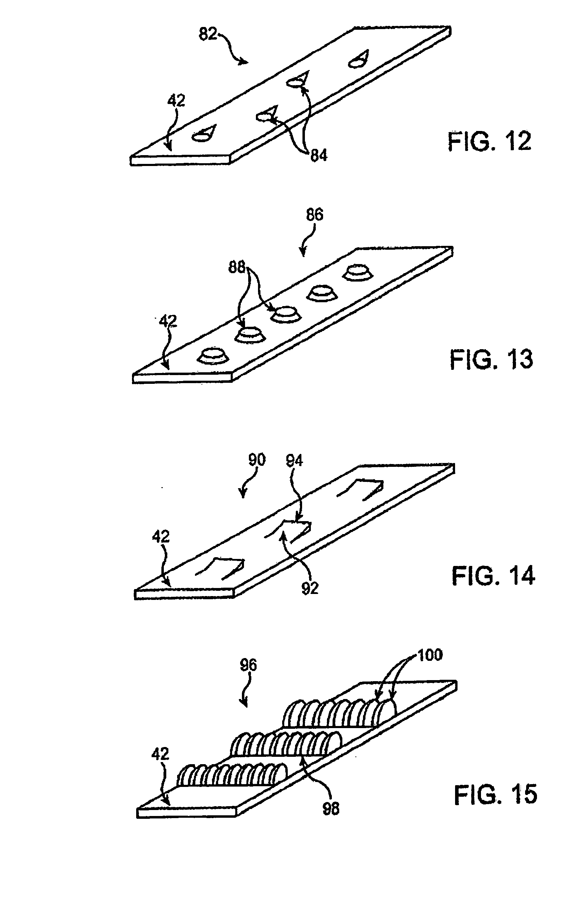

Referring to Fig. 12, in another alternative embodiment, a flexible tissue modification device 82 may include multiple tissue modifying members 84 formed as holes in substrate 42 with raised edges, such as are found on a cheese grater. The raised edges of cutting members 84 may be sharp, to provide cutting. Any number of tissue modifying members 84 may be included, they may have any desired size, and they may be formed on substrate in any pattern. In some embodiments, cut tissue may pass through the holes of cutting members 84 and thus through substrate 42. In some embodiments, a tissue capture device or member may be coupled with the back side of substrate 42 to collect cut tissue that passes through cutting members 84.

Referring to Fig. 13, in another alternative embodiment, a flexible tissue modification device 86 may include multiple tissue modifying members 88 formed as upward-facing holes in substrate 42. The raised edges of cutting members 88 may be sharpened, to provide cutting. Any number of tissue modifying members 88 may be included. In some embodiments, cut tissue may pass through the holes of cutting members 88 and thus through substrate 42. In some embodiments, a tissue capture device or member may be coupled with the back side of substrate to collect cut tissue that passes through cutting members 88.

Referring to Fig. 14, in another alternative embodiment, a flexible tissue modification device 90 may include multiple tissue modifying members 92 formed as raised flaps in substrate 42, with each flap 92 including a sharpened cutting edge 94. Any number of tissue modifying members 92 may be included. In some embodiments, cut tissue may pass underneath the flap-like cutting members 92 and thus through substrate 42. In some embodiments, a tissue capture device or member may be coupled with the back side of substrate to collect cut tissue that passes through cutting members 92.

Referring to Fig. 15, in another alternative embodiment, a flexible tissue modification device 96 may include multiple tissue modifying members 98 formed as rounded cutting devices coupled with substrate 42. In one embodiment, each cutting member 98 may include multiple ridges, divided by grooves. In one embodiment, cutting members 98 may have a spiral or screw-like configuration.

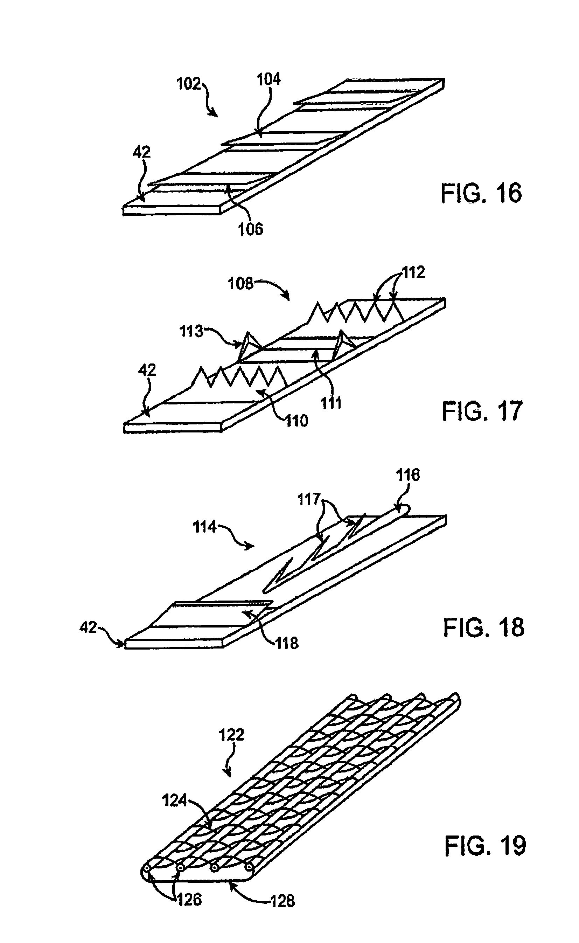

Referring to Fig. 16, in another alternative embodiment, a flexible tissue modification device 102 may include multiple tissue modifying members 104 comprising thin, flap-like blades coupled with substrate 42, each cutting member 104 including a sharp blade edge 106. Any number, size and configuration of blades may be used.

Referring to Fig. 17, in another alternative embodiment, a flexible tissue modification device 108 may include multiple different types of tissue modifying members 110, 111. For example, one embodiment may include one or more jagged tissue cutters 110 each having multiple, triangular, raised teeth 112, and one or more bladed tissue cutters 111, each having multiple blades 113. Teeth 112 and/or blades 113 may be configured specifically to cut ligamentum flavum tissue, bone, or both, in various embodiments.

Referring to Fig. 18, in another alternative embodiment, a flexible tissue modification device 114 may include substrate 42, a tissue engaging member 116 including multiple barbs 117 (or hooks, needles or the like), and one or more tissue cutting members 118, such as a raised blade. In various embodiments, tissue engaging member 116 may be configured to hook, snag, grab or otherwise engage soft tissue, such as ligamentum flavum, and pull or stretch such tissue as it is pulled or pushed across the tissue. Tissue cutting member 118 may follow behind tissue engaging member 116 and cut the stretched/pulled tissue. Such stretching or pulling of tissue before cutting may facilitate or enhance tissue cutting.

Referring to Fig. 19, in another alternative embodiment, a flexible tissue modification device 122 may include a wire mesh 124 coupled with multiple supporting structures 126 and an atraumatic material 128 on one side. All components may be made of any suitable material, such as those listed previously.

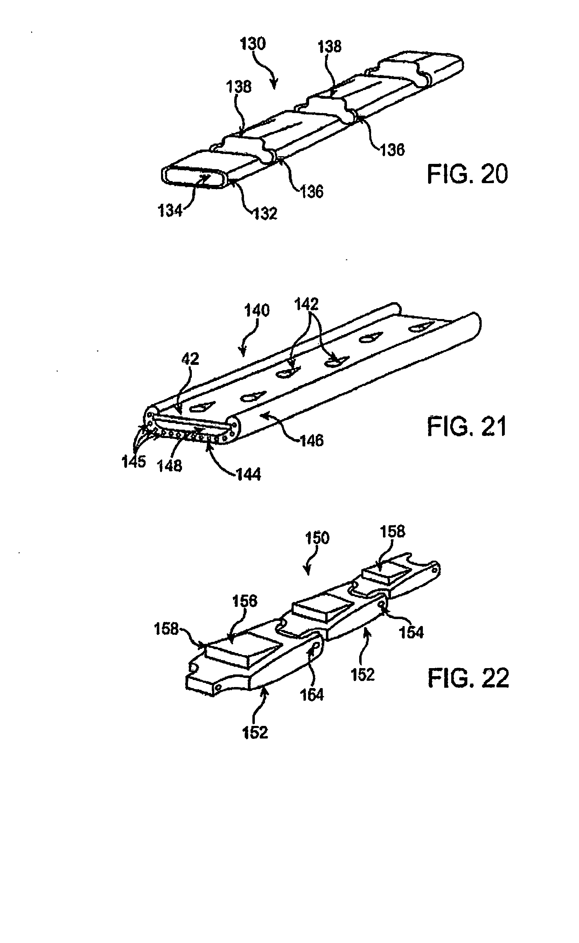

Referring to Fig. 20, in another alternative embodiment, a flexible tissue modification device 130 may comprise a hollow, flattened shaft 132, having central chamber or lumen 134, into which multiple grooves 136 may be cut. An edge of each groove 136 may be raised and sharpened to form a blade edge 138, thus forming a multiple, bladed tissue modifying members. Tissue cut by blades 138 may pass under blades 138 to collect within lumen 134 and may thus be transported out of the patient.

Referring to Fig. 21, in another alternative embodiment, a flexible tissue modification device 140 may include multiple tissue modifying members 142 formed as holes in substrate 42 with raised edges, such as are found on a cheese grater. The raised edges of cutting members 142 may be sharpened, to provide cutting. Any number of tissue modifying members 142 may be included. In some embodiments, cut tissue may pass through the holes of cutting members 142 and thus through substrate 42. In some embodiments, a tissue collection member 144, forming a tissue collection chamber 148, may be coupled with the back side of substrate 42 to collect cut tissue that passes through cutting members 142. Tissue collection member 144 may also serve as an atraumatic tissue protection member and may include, for example, side bumpers 146 to avoid damaging non-target tissue with sharp edges of device 140. In some embodiments, tissue collection member 144 may be strengthened by multiple fibers 145, such as wires or carbon fibers.

Referring to Fig. 22, in another alternative embodiment, a flexible tissue modification device 150 may include multiple sections 152 linked together via linkages 154 to form a flexible device configuration analogous to that of some watch bands. A tissue modifying member 156 having a cutting edge 158 may be disposed on one side of each section 152 to cut tissue.

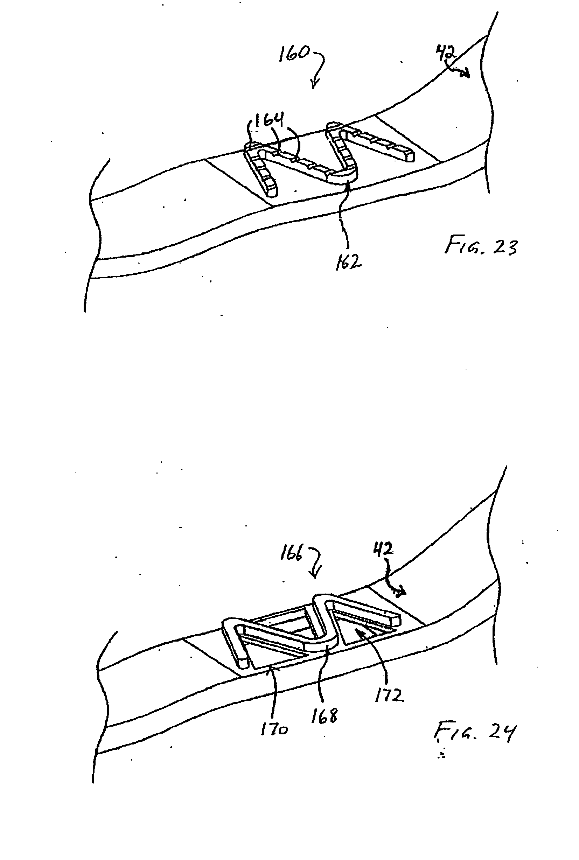

Referring to Fig. 23, in another alternative embodiment, a flexible tissue modification device 160 may include one curved tissue modifying member 162 having multiple ridges.

Referring to Fig. 24, in another alternative embodiment, a flexible tissue modification device 166 may include one curved tissue modifying member 168 and multiple apertures 170 in substrate 42, each aperture opening into a tissue collection chamber 172 in substrate.

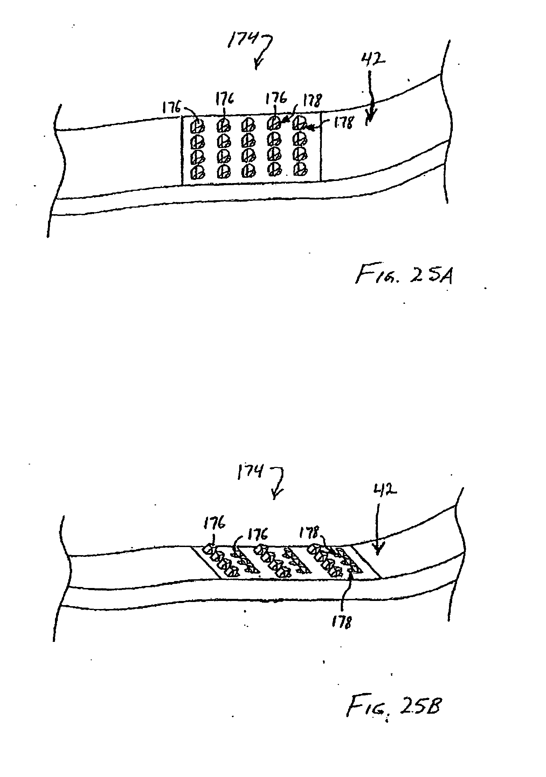

Referring to Fig. 25A and 25B, in another alternative embodiment, a flexible tissue modification device 174 may include multiple, raised tissue modifying members 176, each disposed on substrate 42 adjacent an aperture 178, through which cut tissue may pass into a tissue collection chamber.

In various embodiments, any given flexible tissue modification device may act on tissue in a number of different ways, such as by cutting, ablating, dissecting, repairing, reducing blood flow in, shrinking, shaving, burring, biting, remodeling, biopsying, debriding, lysing, debulking, sanding, filing, planing, heating, cooling, vaporizing, delivering a drug to, and/or retracting target tissue. For example, many of the devices described above may also optionally be loaded with a drug, bone wax, gel foam, or the like, which may be deposited during a tissue modification procedure. Any suitable drug may be delivered via the devices in various embodiments, such as but not limited to thrombin, NSAID, local anesthetic or opioid. In some embodiments, devices may also deliver an implant, such as a stent-like implant for maintaining patency of decompressed intervertebral foramen, a rivet, staple or similar device for retracting ligamentum flavum tissue, a tissue dressing, or the like. In some embodiments, devices may cool or freeze tissue for analgesia or to change the tissue's modulus of elasticity to facilitate tissue modification. Some embodiments of devices may also include a visualization and/or diagnostic component, such as an ultrasound, MRI, reflectance spectroscopy, fiber optic, endoscope, charge-coupled device (CCD), complementary metal-oxide semiconductor (CMOS) or other device.

Any of the devices described herein may also optionally include one or more components for neural identification and/or localization. For example, in some embodiments, a flexible tissue modification device may include one or more nerve stimulation electrodes on a backside or underside of the device (i.e., a side designed to be atraumatic and face non-target tissue). The electrode(s) may be used to confirm that the atraumatic side of the device is in contact with non-target neural tissue, thus also confirming that the tissue modification members of the device are facing target tissue. In some embodiments, the devices may also include one or more electrodes on an upper surface, at or near the tissue modification members, to further confirm a desired placement of the device.

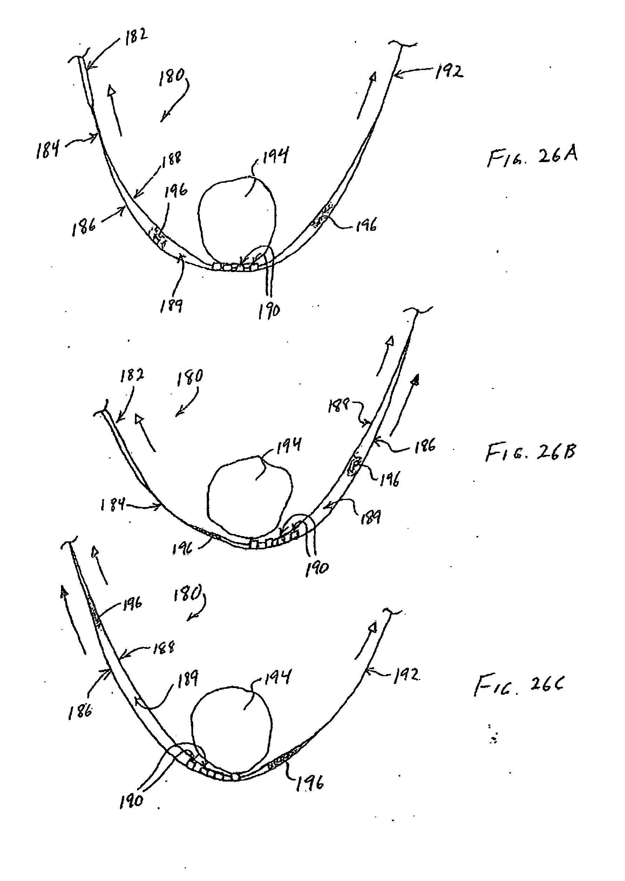

In various alternative embodiments, any of the tissue modification devices and method described above may be used in combination with one or more vertebral distraction devices. In one embodiment, for example, an interspinous implant such as the X STOP™ implant (offered by St. Francis Medical Technologies, Inc., Alameda, Calif., www.sfmt.com) may be inserted between adjacent vertebrae, and then access devices and/or tissue removal devices described herein may be used to remove or otherwise modify spinal tissue. Such an implant may be inserted and left in place after a procedure, while in alternative embodiments a distraction device may be used only during a tissue removal procedure. With reference now to Figs. 26A-26C, one embodiment of a flexible tissue modification device 180 and method for using it to remove tissue are demonstrated. As with previously described embodiments, device 180 may be used in an epidural space and intervertebral foramen of a spine to treat spinal stenosis but is shown in Figs. 26A-26C in diagrammatic form, acting on a generic piece of tissue. In the embodiment shown, tissue modification device includes a rigid proximal shaft portion 182 and a flexible distal shaft portion, the latter of which may be coupled with a guidewire 192 during use. Flexible distal shaft portion 184 may include a lower substrate 186 and an upper substrate 188, with a tissue collection space formed between the two and with multiple tissue modifying members 190 being coupled with the lower substrate 186 so as to extend through one or more apertures in upper substrate 188. Device 180 may be tensioned (hollow-tipped arrows) and reciprocated (Figs. 26B and 26C, solid-tipped arrows) to move cutting members 190 back and forth across tissue and thus remove the tissue.

In one embodiment, as tissue is removed 196, it may pass through the aperture(s) in upper substrate 188 and become trapped in tissue collection area 189 between substrates 186, 188. As device 180 is reciprocated back and forth under tension, trapped tissue 196 may be squeezed between substrates to move farther and farther away from cutting members 190, thus allowing for more cut tissue 196 to be passed into and moved through collection area 189. In some embodiments, device 180 may further include side enclosures disposed between upper substrate 188 and lower substrate 186 to prevent cut tissue 196 from exiting out the sides of collection area 189. Upper substrate 188 may also help protect non-target tissues from harm, such as lateral vessels supplying a facet joint with blood supply.

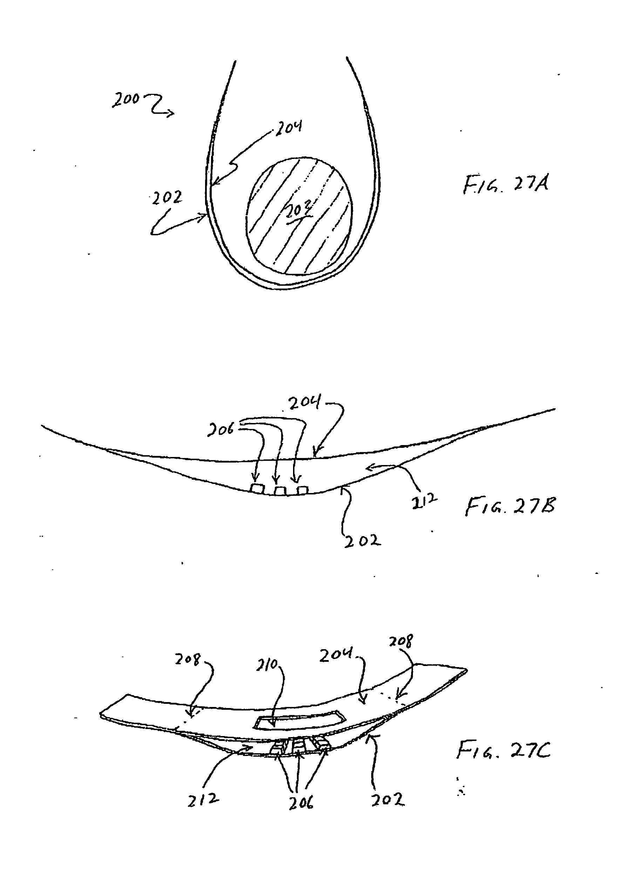

Figs. 27A-27C illustrate another embodiment of a two-substrate, flexible tissue modification device, similar to that described in reference to Figs. 26A-26C. Fig. 27A demonstrates a method of making a device 200 by coupling an upper substrate 204 to a lower substrate 202 while the two are wrapped around a round structure, such as a dowel rod 203. By wrapping substrates 202, 204 around a dowel and then attaching them to one another at attachment points, upper substrate 204 will have a smaller radius of curvature than lower substrate 202. As is seen in Figs. 27B and 27C, when device 200 is then straightened, lower substrate 202 bows outward relative to upper substrate 204, thus forming a space 212 for tissue collection between the two. Additionally, when tissue modifying members 206 are attached to the top side of lower substrate 202, upper substrate 204 will rise above the tops of cutting members 206 when device 200 is held in a straight configuration, but cutting members 206 will protrude through one or more apertures 210 in upper substrate 204 when device 200 is held in a curved configuration. In various embodiments, substrates 202, 204 may be coupled together at attachment lines 208, which may be located at any desired distance from cutting members 206.

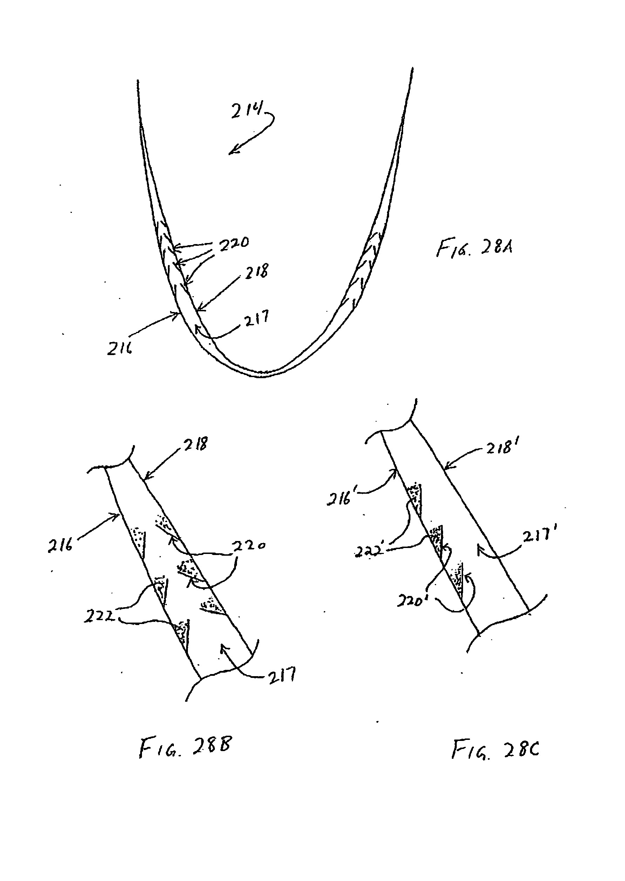

Referring now to Figs. 28A and 28B, another alternative embodiment of a double-substrate, flexible tissue modification device 214 is shown. This embodiment is much like the embodiment just described in reference to Figs. 27A-27C, including an upper substrate 218 attached to a lower substrate 216 to form a tissue collection area 217 between the two. This embodiment include an additional feature, however, of multiple, uni-directional valves 220, which help to direct cut, collected tissue away from tissue modifying members (not shown) of device 214. As shown in Fig. 28B, which shows a magnified portion of Fig. 28A, cut tissue 222 may be trapped by valves, thus preventing it from moving toward the middle portion of tissue collection chamber 217 and toward the tissue modifying members.

Fig. 28C shows a portion of an alternative embodiment of a device, including an upper substrate 218', a lower substrate 216', a tissue collection chamber 217', and multiple one-leaf valves 220' for trapping cut tissue 222'.

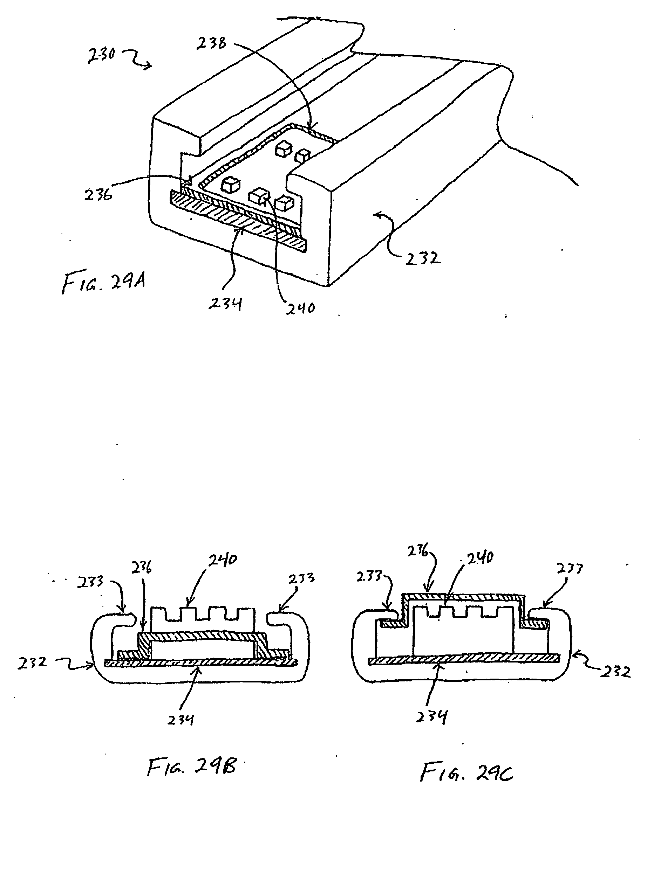

With reference now to Fig. 29A, in another embodiment, a flexible portion of a tissue modification device 230 may include a shaft 232, a substrate 234 disposed in shaft 232, an upper floating substrate 236 disposed over substrate 234 and including an aperture 238, and multiple tissue modifying members 240 disposed on substrate 234 and extending through aperture 238.

Figs. 29B and 29C are end-on views of a slightly varied embodiment, in which floating substrate 236 is shaped, and tissue modifying members 240 are more raised. These figures demonstrate that floating substrate is free to float downward, as in Fig. 29B, so that cutting members 240 extend through aperture 238 to cut tissue, and is also free to float upward, as in Fig. 29C, so that it covers tissue modifying members 240 and prevents them from cutting tissue. As floating substrate 236 floats upward, it is trapped by arms 233 of shaft 232. As tissue is cut by cutting members 240, it may pass through aperture 238 to reside in the space between substrate 234 and floating substrate 236. As more cut tissue is cut and collected in the space, floating substrate 236 may rise and cutting members 240 may be less and less exposed to cut tissue. Thus, floating substrate may help determine when a sufficient amount of tissue has been removed during a procedure.

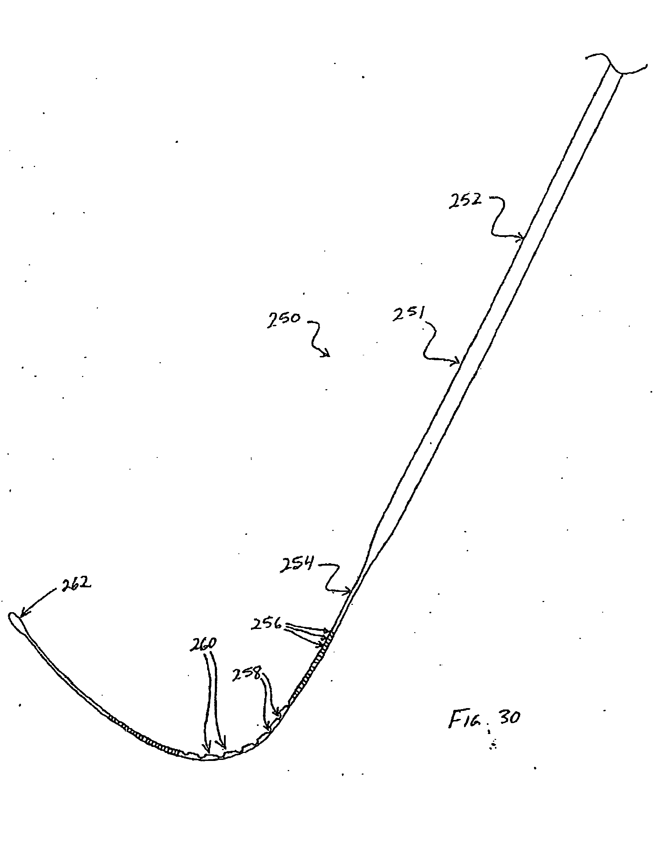

Referring now to Fig. 30, in some embodiments, a flexible tissue modification device 250 may include a hollow shaft 251, a proximal end of which may be coupled with a proximal handle (not shown) and a distal end of which may include a guidewire coupler 262. In one embodiment, shaft 251 may be formed from one piece of material, such as a hollow tube of stainless steel, and guidewire coupler 262 may also be formed from the same piece-of material or, alternatively, may be a separate piece welded to shaft 251. Shaft 251 may include a rigid proximal shaft portion 252 and a distal flexible shaft portion 254. In some embodiments, shaft 251 may have an approximately tubular shape at its proximal portion 252 and may be flattened to form distal portion 254. Distal portion 254 may include multiple slits 256 to confer flexibility or added flexibility. Distal portion 254 may also include multiple cutting members 260, formed by creating grooves 258 in distal portion 254 and raising cutting edges at one side of each groove 258, as in the embodiment described in Fig. 20. In various embodiments, any number of grooves, 258, cutting members 260 and slits may be included. Different embodiments may include different numbers of these features, for example, depending on tissue to be cut, anatomical structures to be accessed and the like.

In some embodiments, device 250 may include means for transporting removed tissue through the device, either to facilitate storage of the removed tissue in another part of the device, to transport the removed tissue out of the patient, or both. Other device embodiments may also include tissue transport means, such as the embodiments described in relation to Figs. 26-29, which include multiple substrates that form tissue collection chambers between the substrates. In some embodiments, two-substrate devices such as those in Figs. 26-29 may be at least partially covered with material or attached at the sides, to form an enclosed tissue collection chamber between the substrates. In other embodiments, tissue may be moved between the two substrates even though the tissue collection area is not fully enclosed. In any event, some embodiments of a flexible tissue modification device that include means for collecting tissue may also optionally include means for transporting the tissue out of the device and, thus, out of the patient. In some embodiments, cut tissue may simply be collected in one or more tissue collection chambers or storage areas and then may be removed from the patient by removing the device from the patient. In other embodiments, however, tissue transport means may be used to move tissue out of the patient through a tissue modification device without removing the device from the patient. Several embodiments of such tissue transport means will now be described.

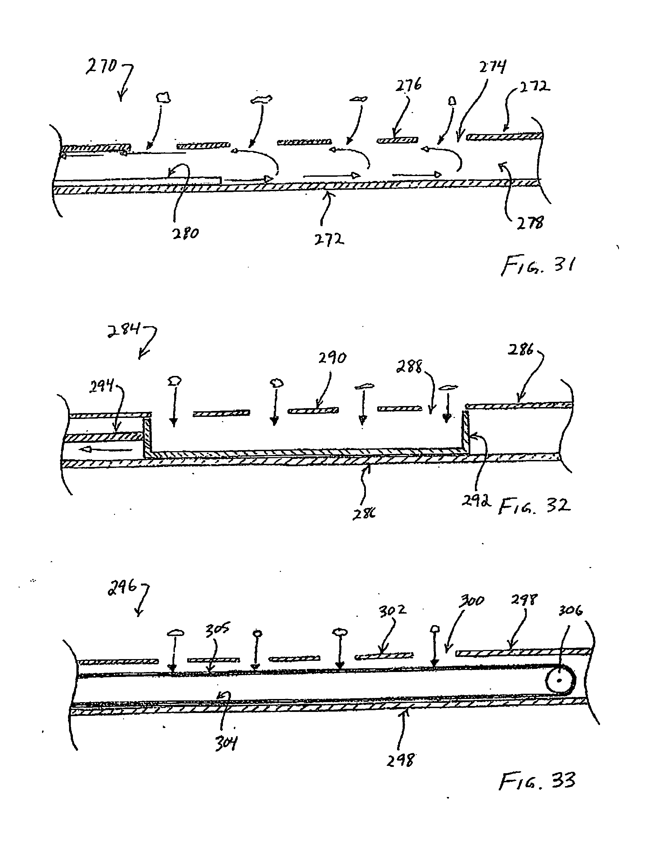

Referring now to Fig. 31, in one embodiment, a flexible tissue modification device 270 (shown diagrammatically in side cross-section) may include a hollow shaft 272, forming an inner tissue collection lumen (or "chamber") 278. Grooves 274 may be formed in a surface of shaft 272, and tissue modifying members 276 may be formed between grooves 274. (For ease of illustration, cutting members 276 are shown in diagrammatic form and are not raised or sharpened, although in some embodiments one edge of each cutting member 276 may be raised and sharpened.) In one embodiment, tissue transport means may include an irrigation tube 280 (or lumen) for introducing fluid into tissue collection lumen 278, suction (or "vacuum") force may be applied to tissue collection lumen 278 to suction fluid and cut tissue out through device 270. In an alternative embodiment, a separate suction tube (or lumen) may be included (not shown). As tissue is cut by tissue modifying members 276, it may be directed into collection lumen 278 (solid-tipped arrows) via suction force as well as by the force of circulating irrigation fluid. In various embodiments, any known suction device(s) may be coupled with a proximal end of device 270 to provide suction, and any known irrigations device(s) may be coupled with irrigation tube 280 to provide irrigation. Irrigation tube 280 and collection lumen 278 may have any desired diameter and be made of any suitable material(s).

With reference now to Fig. 32, in an alternative embodiment, a flexible tissue modification device 284 may include a hollow shaft 286 including multiple grooves 288 and tissue modifying members 290, and tissue transport means including a moveable tissue collection compartment 292 disposed within shaft 286, and a pull wire 294 to pull compartment 292 out of device 284. As tissue is cut, at least some of it may fall into (or is directed by tissue modifying members 290 into) tissue collection compartment 292. Pull wire 294 may be used at any time to retract compartment 292 proximally through device 284 to transport cut tissue out. In some embodiments, compartment 292 may be emptied of cut tissue and advanced back into shaft 286, such as by using pull wire 294 to push it into shaft 286. In another embodiment, compartment 292 may be filled only once during a procedure. Compartment 292 may have any desired size, shape and configuration, according to various embodiments, and may be made of any suitable material.

In another alternative embodiment, and referring now to Fig. 33, a flexible tissue modification device 296 may include a hollow shaft 298 including multiple grooves 300 and tissue modifying members 302, and tissue transport means including a flat piece of flexible material 304, having an outer, tissue-adhering surface 305 and being disposed over a rotating dowel 306, such as in a conveyor belt configuration. As cut tissue enters or is directed into shaft 298 through grooves 300, it may stick to adhering surface 305 and thus be conveyed out of device 296 proximally. In some embodiments, dowel 306 and/or a proximal dowel (not shown) may have a ratcheting mechanism, so that material 304 can only move in one direction. Any material, such as various flexible polymers and the like, may be used for making material 304 in various embodiments.

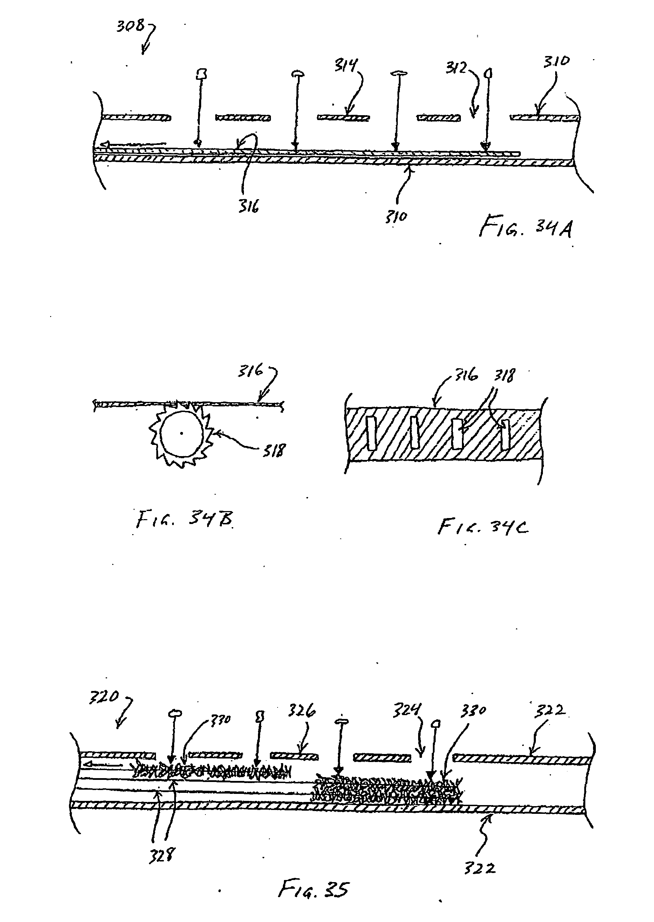

Referring to Fig. 34A, in another embodiment, a flexible tissue modification device 308 may include a hollow shaft 310 including multiple grooves 312 and tissue modifying members 314, and tissue transport means including a retractable tissue adhering material 316. As with the above embodiment, any suitable material 316 may be used. In some embodiments, material 316 may be retracted, cleaned and reinserted. In other embodiment, multiple pieces of material 316 may be used.

In some embodiments, as show in Figs. 34B and 34C, a ratcheting mechanism 318 may be used to retract material 316. For example, material 316 may include multiple apertures 318 or slits, which teeth of ratcheting mechanism 318 may used to pull material 316 out of device 308.

With reference to Fig. 35, in another alternative embodiment, a flexible tissue modification device 320 may include a hollow shaft 322 including multiple grooves 324 and tissue modifying members 326, and tissue transport means including multiple wires 328, each having a tissue adhering material 330 attached thereto or formed therein. For example, material 330 and wire 328 may be configured similar to a pipe cleaner. In another embodiment, material 330 may comprise multiple bends, hooks or other patterns bent into wires 328. In various embodiments, as few as one wire 328 or as many as twenty or more wires 328 may be preloaded into device 320. Where multiple wires 328 are preloaded, they may be withdrawn one by one during a procedure to remove tissue as it is cut. In some embodiments, one or more wires 328 may be inserted during a procedure-i.e., new wires 328 may be inserted and/or used wires 328 may be cleaned and reinserted. Any suitable size, shape, configuration, number and material(s) may be used, according to various embodiments.

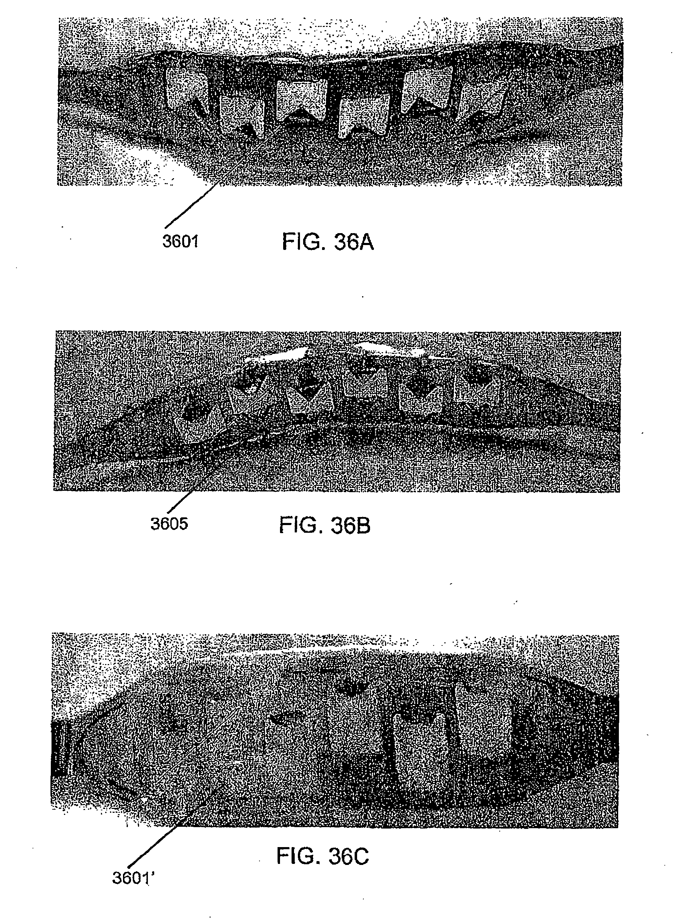

FIG. 36A-36B show another variation of a tissue modification device having a tissue collection region (pouch 3601). In this example, the tissue collection region is configured as a pouch that is formed between the bottom (the back surface shown in FIG. 36A) and the top (the cutting surface 3605 shown in FIG. 36B). The bottom surface forming the pouch (the back surface of the device) may be formed of a flexible material. In this example, the back surface is formed of a flexible polymeric material such as PET. The cutting surface may be referred to as the first major surface in this example (FIG. 36B) and the back surface may be referred to as the second major surface, which is the outer side of the pouch, shown in FIG. 36A. In FIGS. 36A and 36B, the first major (e.g., substantially flat) surface and the second major surfaces are attached to each other by stitching. For example, a Nitinol thread may be used to stitch the material forming the second major surface to the material (metal) forming the first major surface. Alternatively, the second surface could be bonded to the first surface through adhesives and/or heat processes used to fuse the two fuse the two surfaces. Also, the second surface could be tied to the first surface through an injection molding process.

FIG. 36C shows another variation of a tissue modification device including a tissue collection region configured as a pouch 3601'. This example is very similar to the example shown in FIG. 36A, except the stitching used to secure the materials forming the first and second major surfaces together are stitched differently. In some variations the tissue collection region is removable. For example, the material forming the second major surface may be removable. In some examples, the tissue collection region may be expandable.

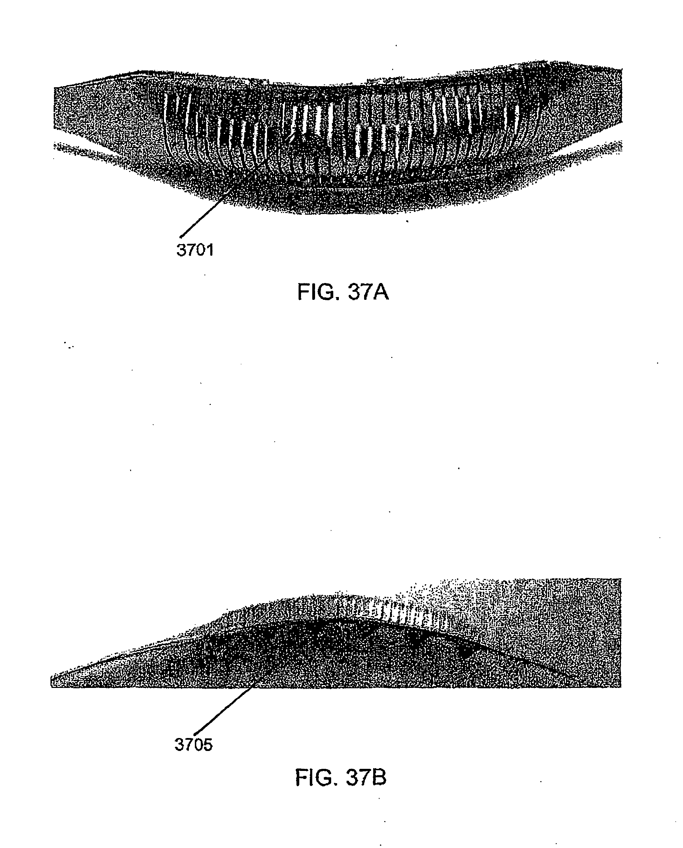

FIGS. 37A and 37B illustrate another variation of a tissue modification device in which the second major surface (the back surface in FIG.37A) and the first major surface (the cutting surface in FIG. 37B) form a tissue collection region between them. In this example, the second major surface is formed of a metallic material that is cut in multiple lines. The first and second major surfaces may fabricated from a single tubular element. These parallel cuts form slats, and they may allow the metallic bottom region 3701 to be more flexible, while maintaining the separation of the tissue collection region, based on the relative rigidity of the metal 3701 forming the bottom surface. Thus, the tissue collection region formed between the bottom surface 3701 and the cutting surface 3705 may be held open even when the device is in operation. In some variations the tissue collection region includes a frame or structure that helps to hold the tissue collection region open even when tension is applied to drive the device against the tissue, in order to cut or otherwise modify the tissue.

Any of the tissue collection regions described herein may be configured as static tissue collection regions. A static tissue collection region allows storage of the collected tissue within the region, rather than removal from the device. For example, in some variations the tissue collection region is a pouch (such as a removable pouch). During the procedure, tissue cut by the device can be stored in the pouch (static storage). The tissue collection region can later be emptied, or the entire pouch can be disposed of.

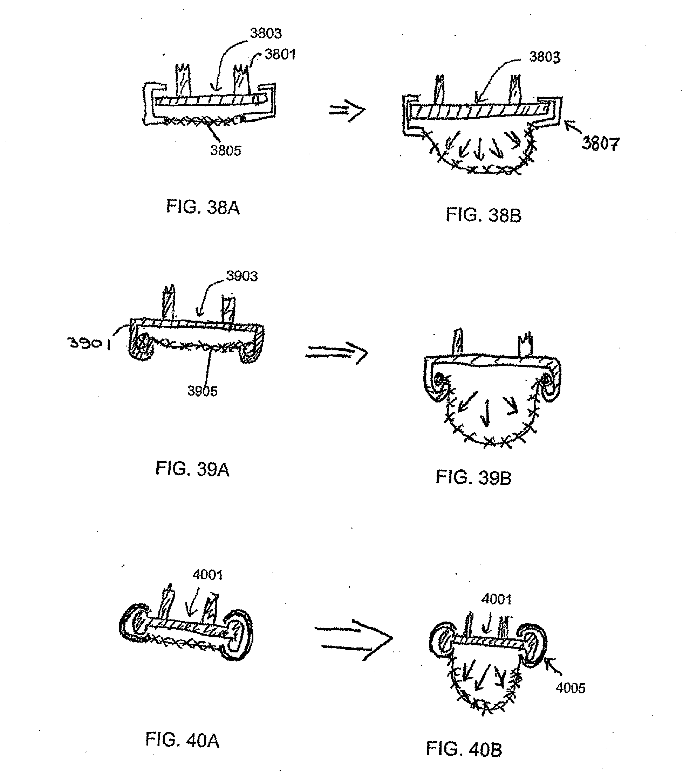

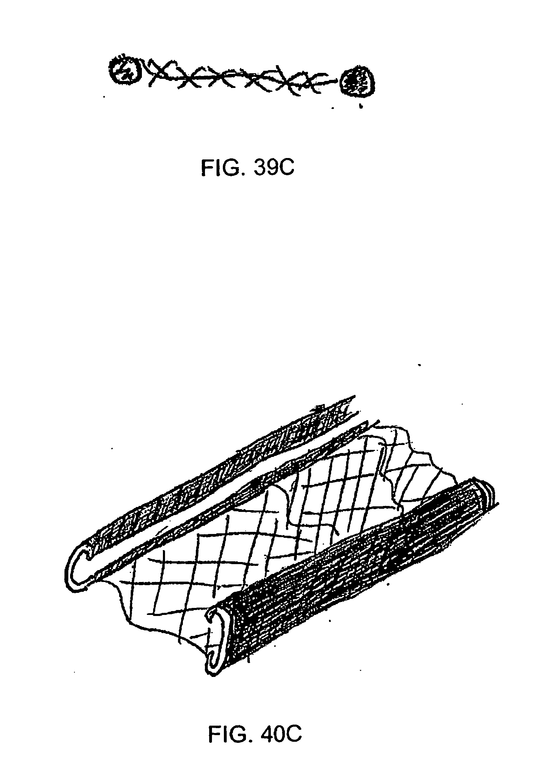

Removable tissue collection regions (e.g., removable pouches) and tissue modification devices including removable tissue collection regions are illustrated in FIGS. 38A-44C. For example, FIG. 38A and 38B illustrate the operation of an expandable and removable tissue collection region. In this example, the removable pouch is formed by a flexible region 3805 that is connected to two slideable tracks 3807 that are configured to mate with the upper (cutting) surface 3803, including blade 3801. As shown in FIG. 38B, during use the tissue collection pouch fills with material and expands. The pouch may be removed (e.g., when sufficiently full) and emptied (for reuse) or disposed of. Similar variations are shown in FIGS. 39A-40B. For example, in FIG. 39A-39B, the cutting surface 3903 (the first major surface) includes a track or guide region 3901 into which the expandable and removable second major surface 3905 slides to form the pouch region (tissue collection region) between the cutting surface and the removable surface. FIG. 39C illustrates a cross-section through just the removable surface. In this example, the expandable material forming the surface 3905 can be bonded to two parallel rods or wires that are secured in the track connected to the cutting surface.

FIGS. 40A and 40B show a similar expandable tissue collecting region in which the track or guide 4005 is attached to the second (expandable) surface. In this example, the second, expandable, surface is removable, but can slideably engage the upper (cutting 4001) surface. FIG. 40C shows a perspective view of the lower surface.

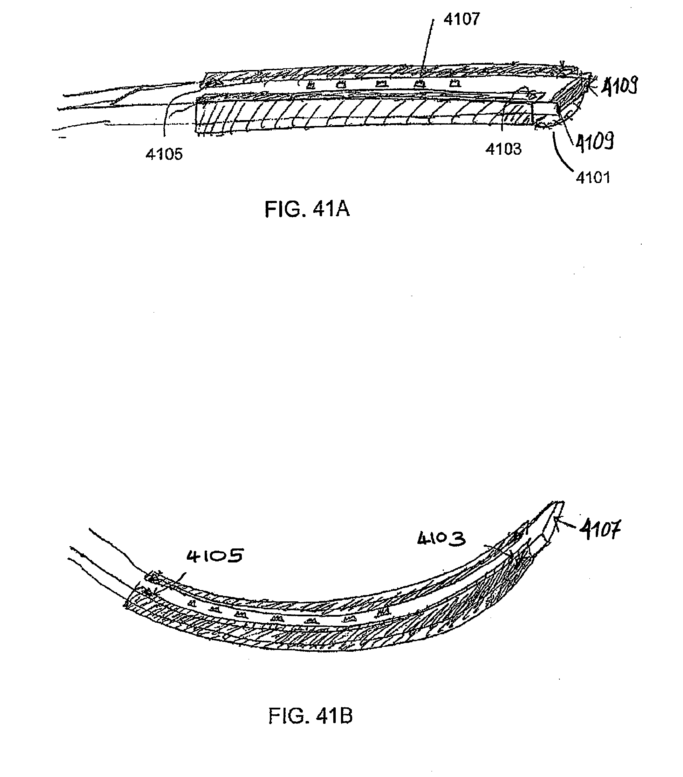

FIGS. 41A and 41B show another variation of an expandable and removable tissue collection region formed between a first and second major surface, similar to the variation shown in FIGS. 38A and 38B. In this example, the lower surface (including the expandable member 4101) includes two tracks 4109 that can mate with the cutting surface 4107 so that the two surfaces can slide together to form the tissue collection region therebetween. A pouch is formed between these two surfaces. The lower surface may be secured to the upper surface by engaging locking tabs 4103, 4105 between the two surfaces. Locking tabs may be any appropriate engagement region between the two surfaces. For example, the locking tabs may be indentations on one or both surfaces that engage with a projection on the opposite surface. FIG. 41B illustrates the device of FIG. 41A during flexion. Bending the device may help secure the two surfaces together. For example, bending the device may place the tab locks in tension and/or compression, helping to secure the cutting surface 4107 and the lower, expandable surface together. In this example, the lower surface slides onto the distal end of the cutting surface.

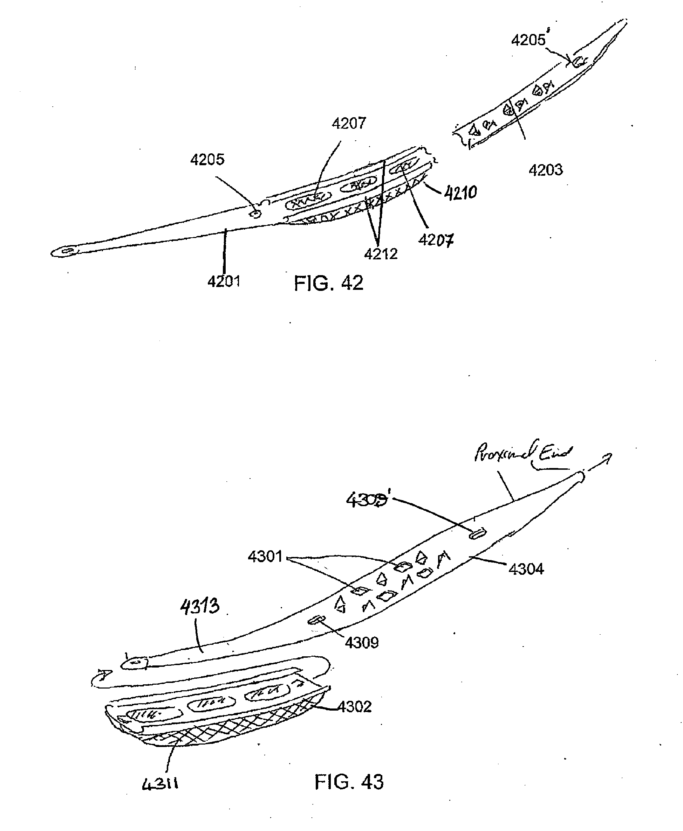

In some variations, the lower surface forming the tissue collection region is disposable, so that after use (e.g., after filling with tissue) it may be discarded and the cutting surface 4203 may be re-used. FIG. 42 illustrates one variation of a device having a disposable member 4201. The disposable member in this example has two parallel surfaces. The first surface includes slots 4207 or passages which open into a pouch formed between this first surface and a second expandable material 4210. The disposable tissue collection region mates with the cutting surface 4103 through slots 4212 on the upper surface of the disposable tissue collection region. The disposable tissue collection region (pouch) may be secured to the cutting surface by lock tabs 4205, 4205'.

FIGS. 43-44C are other variations of removable (and possibly disposable) tissue collection pouches that mate with a (possibly reusable) cutting surface. For example, in FIG. 43, the disposable and removable pouch region 4302 is configured to slide over the cutting surface 4304. The cutting surface includes a plurality of debris ports 4301. When the waste pouch 4302 is positioned and secured to the cutting surface 4304 (e.g., by engaging lock tabs 4309, 4309'), these debris ports 4301 align with the slots opening into the tissue collection region 4311. In this example, the waste pouch may be slid over the distal end 4313 of the cutting surface.

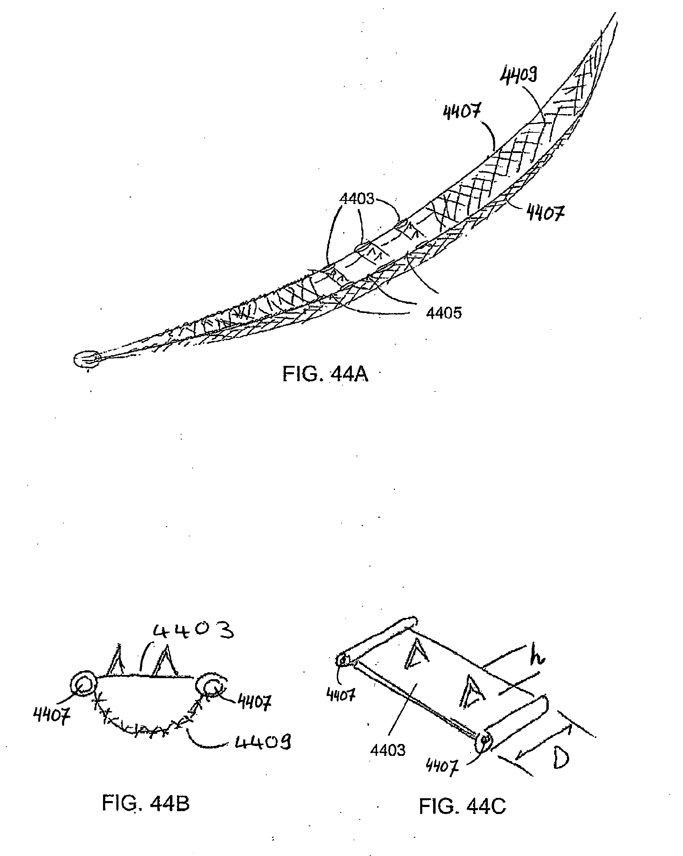

FIG. 44A-44C illustrate a tissue modification device according to the present invention in which the device is formed from at least 2 wires 4407, cutting surface 4403 and a mesh 4409 attached to the wires. The cutting surface is actually multiple discrete cutting surfaces 4403 that are connected to the two wires. The mesh is attached to the wires and forms a tissue collection region between the upper surface holding the cutting surface(s) and the lower (protective) surface. In FIG. 44A three cutting surfaces are shown, and FIG. 44C shows an enlarged view of one of the discrete cutting surfaces 4403. The cutting surface includes multiple cutting surfaces or blades that project upward to a height, h, and the cutting surface is some minimum width, D, as shown in FIG. 44C. FIG. 44B shows a cross-section through the tissue modification device shown in FIG. 44A at the level of a cutting surface. Between the cutting surfaces, the upper surface of the device (the surface that will be driven against the tissue) includes multiple openings or ports 4405 into the tissue collection region. In some variations, the mesh or wires are formed at least partially of a shape memory material, such as a Nitinol. For example, the framework may be made of Nitinol wire. In some variations, the mesh forming the device is at least partially made of an expandable material. Thus, the tissue collection pouch may be expandable. The mesh forming the device may be coated and/or impregnated with a lubricious material, such as a lubricious polymer, which will reduce friction when the device is drawn against the target tissue.

Any of the devices described herein may also optionally include one or more components for neural identification and/or localization. For example, in some embodiments, a flexible tissue modification device may include one or more nerve stimulation electrodes on a backside or underside of the device (i.e., a side designed to be atraumatic and face non-target tissue). The electrode(s) may be used to confirm that the atraumatic side of the device is in contact with non-target neural tissue, thus also confirming that the tissue modification members of the device are facing target tissue. In some embodiments, the devices may also include one or more electrodes on an upper surface, at or near the tissue modification members, to further confirm a desired placement of the device.

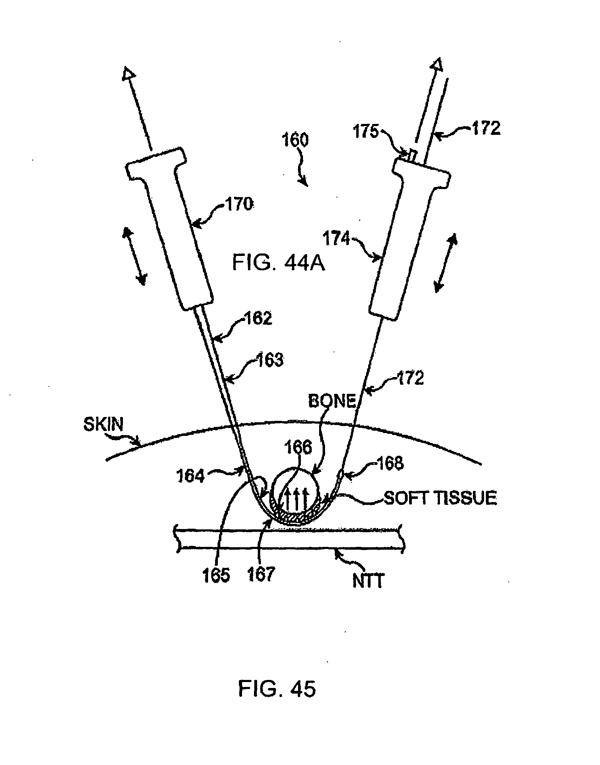

With reference now to FIG. 45, in another alternative embodiment, a tissue modification device 160 may suitably include a proximal handle 170 coupled with an elongate body 162 (or "shaft") having a proximal, rigid shaft portion 163, a distal flexible portion 164 having a first major surface 165 and an opposed second major surface 167, and multiple substantially in-line, substantially vertical blades 166 disposed laterally across first major surface 165. Second major surface 167 may be atraumatic, to inhibit injury to non-target tissues NTT. A guidewire coupler 168 may be formed in (or attached to) flexible portion 164 at or near its distal end, for coupling with a guidewire 172, which in turn may be coupled with a guidewire handle 174 (or "distal handle"), which may include a tightening lever 175 for tightening handle 174 around guidewire 172. In one embodiment, device 160 may have many of the characteristics and be used in much the same way as embodiments described above, such as device 10 of FIG. 2A. The number, height, length, configuration and placement of blades 166, however, may confer unique tissue cutting/removal characteristics to device 160.

In FIG. 45, device 160 is shown passing into a patient, along a curved path between a generic soft tissue/bone combination and nearby non-target tissue NTT, and back out of the patient. In one embodiment, device 160 may be passed into a patient, through an intervertebral space of the patient's spine (between ligamentum flavum and neural/neurovascular tissue), and back out of the patient, as described in detail above with reference to alternative embodiments. Once device 160 is in place for modifying a target tissue, such as soft tissue and/or bone, handles 170, 174 may be pulled (hollow-tipped arrows) to apply force and thus urge blades 166 into soft tissue (single-headed, solid-tipped arrows). Device 160 may then be reciprocated (double-headed, solid-tipped arrows), while maintaining some or all of the pulling force, to remove or otherwise modify the target soft tissue and/or bone. As mentioned previously, before reciprocating device 160 to remove tissue, in some embodiments the device may be used to stimulate nearby nerve tissue, such as with an electrode coupled with second major surface 167 and/or first major surface 167. Such nerve stimulation may help confirm that device 160 has been placed in a desired location for treatment and may be monitored using electromyography (EMG), visual observation of muscle twitch and/or the like. Second major surface 167 may be made atraumatic in a number of different ways, such as but not limited to forming second major surface 167 with an atraumatic material, smoothing surface 167 during the manufacturing process, coupling an atraumatic cover with surface 167 and/or coating surface 167 with a lubricious coating.

In various embodiments, device 160 may be optimized for removal of soft tissue (such as ligamentum flavum or other ligamentous tissue), bone or a combination of both. Such optimization, for example, may be achieved with various heights, lengths, edge types, numbers and/or placement of blades 166. In some embodiments, it may be possible to remove both soft tissue and bone with device 160, such as by continuing to reciprocate device 160 after soft tissue has been removed and/or by using different amounts of pulling force to remove different types of tissue. For example, in one embodiment, if a surgeon only desires to remove soft tissue, he/she may apply a first amount of pulling force. If, instead, the user desires to remove only bone tissue, it may be possible to apply sufficient force to cut immediately through ligament and address bone. In other embodiments, a user may apply a first amount of tension to device 160 to remove soft tissue and a second amount of tension to remove bone, within the same procedure. For example, it typically requires approximately 207 MPa (30,000 psi of force) to cut cortical bone. Thus, in embodiments where it is desired to cut bone, at least some of blades 166 may have bone-cutting tips. In such an embodiment, first major surface 165, when bending over a bone surface, may have an active region with blades 166 that can be urged into soft tissue (such as ligament), and manual tension forces applied to device 160 divided by a combined surface area of the bone cutting tips of blades 166 within the active region may be at least 207 MPa (30,000 psi). In an alternative embodiment, at least some of blades 16 may have bone-protecting ends, and manual tension forces applied to device 160 divided by a combined surface area of the bone-protecting ends of blades 166 within the active region may be less than 207 MPa (30,000 psi). Such an embodiment may facilitate removal of soft tissue, if blades 166 ride or "skate" over the bone and are thus focused on soft tissue removal.

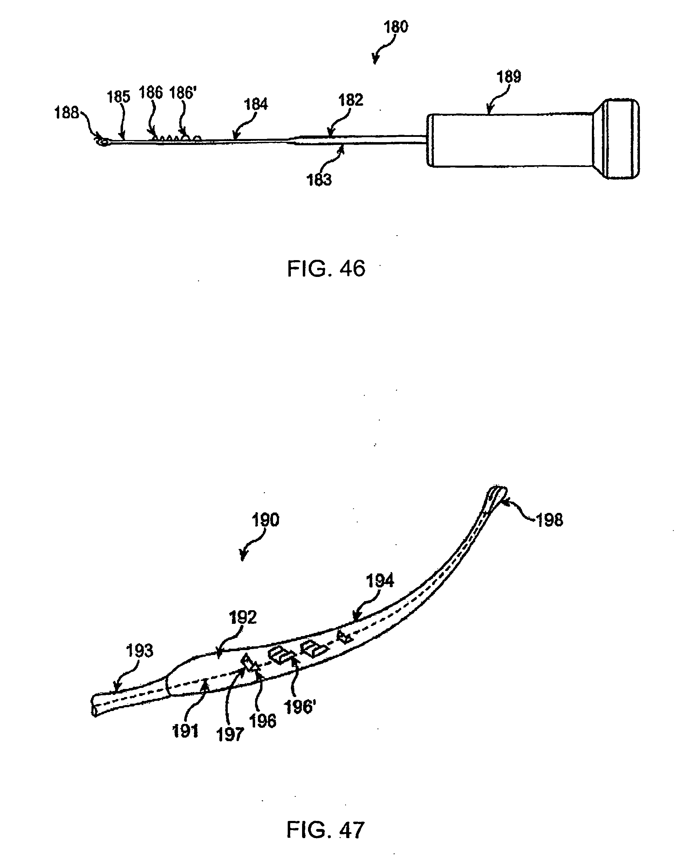

Referring to FIG. 46, in one embodiment a tissue modification device 180 may include a proximal handle 189 coupled with one end of an elongate body 182, which includes a proximal rigid shaft portion 183 and a distal flexible portion 184. Multiple substantially vertical, substantially in-line blades 186, 186' may be disposed on a first major surface 185 of flexible portion 184, while a second major surface 187 approximately opposite first major surface 185 is substantially atraumatic to inhibit damage to non-target tissues during a tissue modification procedure. (Again, by "substantially in-line," it is meant that a side of each blade is aligned at an angle of between about 0 degrees and about 45 degrees relative to the longitudinal axis of the elongate body. By "substantially vertical," it is meant that each blade forms an angle with the first surface of the elongate body of between about 45 degrees and about 90 degrees.) Flexible portion 184 may also include a guidewire coupler 188 at its distal end.

In various embodiments, a number of which are described further below, any suitable combination of blades 186, 186' may be included on a given tissue modification device. For example, device 180 includes four pointed-tip blades 186 and two flat-top blades 186' of various heights and lengths. Various blades may be configured to perform one or more of a number of functions. For example, pointed-tip blades 186 may be ideal for removing bone, while flat-top blades 186' may work best at removing soft tissue and riding along a bone surface, for example to help steer or guide device 180. In some embodiments, all blades on a device may be configured for optimal soft tissue cutting, such as cutting of ligamentum flavum tissue in the spine, while in other embodiments all blades may be configured for optimal bone cutting, such as vertebral bone. Other alternative embodiments may include a combination of blade shapes and configurations to provide multiple different types of cutting. Further discussion of blades combinations and configurations follows below.

With reference now to FIG. 47, an alternative embodiment of a tissue modification device 190 may include an elongate body having a longitudinal axis 191, a rigid shaft portion 193 and a flexible portion 194. Flexible portion 194 may have a lateral axis 195 and may include a guidewire coupler 198 at or near it distal end. In some embodiments, multiple blades 196, 196' may be disposed laterally across a first major surface 192 of flexible portion 194, with each set of two blades 196, 196' extending from a base 197 coupled with surface 192. The embodiment shown includes pointed-tip blades 196 and flat-top blades 196'. In the embodiment shown, and as described in further detail below in relation to an alternative embodiment, some or all blades 196' may be angled, relative to elongate body longitudinal axis 191. Angling blades 196' may cause or facilitate lateral movement of device 190 along a target tissue as device 190 is reciprocated back and forth to modify the tissue, thus providing for wider or more complete tissue modification/removal.

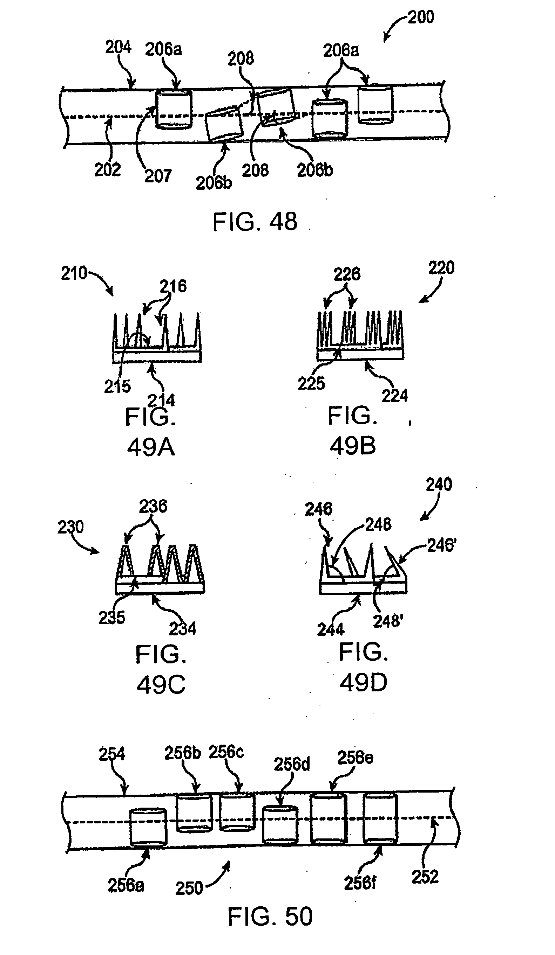

Referring to FIG. 48, a flexible portion 204 of an alternative embodiment of a tissue modification device 200 is shown in top view. In this embodiment, flexible portion 204 has a longitudinal axis 202, and multiple sets of blades 206, each set of two blades extending from an associated base 207, coupled with a first surface of flexible portion 204. The sets of blades 206 may be distributed axially along longitudinal axis 202 and may also be distributed laterally across the first major surface. In the embodiment shown, three blades 206a are aligned such that their sides are approximately in line with longitudinal axis 202, while two blades 206b are angled, such that each side forms an angle 208 with longitudinal axis 202. Again, such angled blades 206b may facilitate lateral movement or "steering" of device 200 along a target tissue such as soft tissue and/or bone. In various embodiments, all blades 206 may form an angle of about 0 degrees relative to longitudinal axis 202 (as with blades 206a), all blades may be angled (as with blades 206b), or device 200 may include a combination of angled and non-angled blades. In some embodiments, each blade side may form an angle of between about 0 degrees and about 45 degrees with longitudinal axis 202 of flexible portion 204. As mentioned previously, such blades 206 may be referred to as being "substantially in-line." In a more preferred embodiment, each blade side may form an angle of between about 0 degrees and about 30 degrees relative to longitudinal axis 202. In various alternative embodiments, any number or combination of blades, having any combination of angles, positions on flexible portion 204 or the like may be used.

In various embodiments, blades may be distributed in any of a number of suitable distances and configurations along the first major surface of flexible portion 204. For example, any number of blades 206 may be used in various embodiments, such as but not limited to between two and eight sets of two blades 206 each. In some embodiments, blades 206 are distributed axially along flexible portion 204 at distances selected to confer a desired amount of flexibility to flexible portion 204. Increased space between the sets of blades, for example, may increase the flexibility of flexible portions 204, while placing the sets of blades closer together along longitudinal axis 202 may decrease flexibility of flexible portion 204.

Referring now to FIG. 49A, one embodiment of a tissue modification device 210 is shown in end-on view at the location of a flexible portion 214 with multiple blades 216 coupled with one side. Each set of two blades 216, in this embodiment, extends from a base 215, and each base 215 is coupled with flexible portion 214. As seen in this figure, in some embodiments some or all blades 216 may be laterally offset, relative to one another, along flexible portion 214. Blades 216 of device 210 are substantially vertical, relative to the surface of flexible portion 214 to which they are attached, and they are also aligned at approximately a 0 degree angle relative to the longitudinal axis of flexible body 214. In device 210, blades form approximately a 90 degree angle with flexible body 214 and approximately a 0 degree angle with the longitudinal axis of flexible body 214.

FIG. 49B shows an alternative embodiment of a tissue modification device 220, again in end-on view, where rows of closely spaced blades 226 are attached together on flexible portion 224, analogous to the way sharks' teeth are aligned in rows in a shark's mouth. In this embodiment, sets of six blades 226 (three on each side) extend from one base 225, and each base 225 is coupled with flexible portion 224.

FIG. 49C shows an alternative embodiment of a tissue modification device 230 with four, flat-top blades 236 aligned at an angle relative to the longitudinal axis of flexible portion 234. In this embodiment, each set of two blades 236 extends from an associated base 235.

FIG. 49D shows another alternative embodiment of a tissue modification device 240, including two blades 246 that form an approximately 90 degree angle 248 with a first major surface of a flexible portion 244 and two blades 246' that form a more acute angle 248' with the first major surface. In various embodiments, the sides of each blade may form an angle with the flexible portion of between about 90 degrees and about 45 degrees, or more preferably between about 90 degrees and about 60 degrees. These angles 248, 248' maybe referred to as "tilt," and in any given embodiment, all blades may be tilted (i.e., all form an angle of less than 90 degrees with the surface), no blades may be tilted (i.e., all form an angle of about 90 degrees with the surface), or some blades may be tilted and others may not, as in FIG. 49D.

Referring now to FIG. 50, as mentioned previously, in some embodiments, a tissue modification device 250 may have a flexible portion 254 including multiple blades 256, some of which may be laterally offset relative to one another and others of which may lie along the same line relative to one another. For example, device 250 includes multiple blades 256, all aligned at approximately 0 degrees relative to a longitudinal axis 252 of flexible portion 254. Blades 256a and 256d lie along the same line, relative to each other, as do blades 256b and 256c. Obviously, blades 256a and 256d are offset, relative to blades 256b and 256c. Blades 256e and 256f lie along the same line relative to one another and are placed close to opposite edges of flexible portion 254. In various embodiments, any combination of lateral placement of blades 256 along device 250 may be used. Offsetting blades 256 relative to one another may facilitate cutting or shredding of soft tissue, for example.

In some embodiments, blades 256 may be shaped and/or axially spaced to facilitate or enhance the collection of cut tissue between blades 256. (By "axially spaced," it is meant the longitudinal spacing along longitudinal axis 252.) In some embodiments, axial spacing of blades 256 may also be optimized to provide a desired flexibility to flexible portion 254.

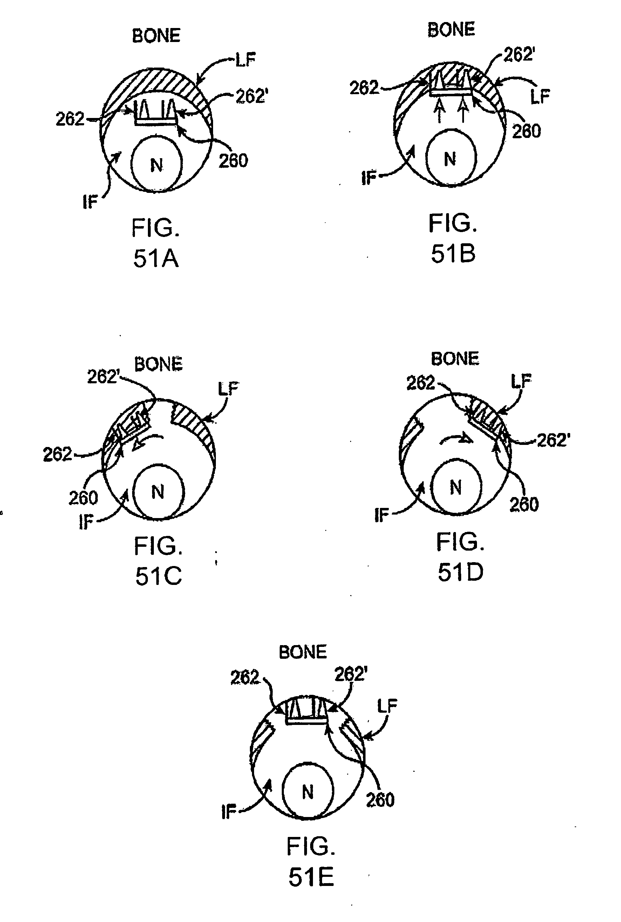

With reference now to FIGS. 51A-51E, a method according to one embodiment is demonstrated for removing tissue using a tissue modification device 260. FIG. 51A is an end-on, diagrammatic representation of an intervertebral foramen IF, showing vertebral bone, ligamentum flavum LF and nerve root N, with device 260 passing through the foramen IF between nerve root N and ligamentum flavum LF. Device 260 may have some blades 262 vertically oriented and at approximately a 0 degree angle relative to the longitudinal axis of device 260, while other blades 262' may be angled, relative to the longitudinal axis.

In FIG. 51B, device 260 has been pulled upward (hollow-tipped arrows) to urge blades 262, 262' into ligamentum flavum LF so that at least one of blades 262, 262' contacts vertebral bone. In some embodiments, some or all of blades 262, 262' may have a height approximately equal to or greater than a thickness of an average ligamentum flavum LF.

Referring to FIG. 51C, when device 260 is reciprocated back and forth along its longitudinal axis, ligamentum flavum LF tissue is removed in one area of the intervertebral foramen IF. As device 260 is reciprocated, angled blades 262' may steer or guide device 260 laterally in the intervertebral foramen IF (hollow-tipped arrow). In some embodiments, for example, device 260 may steer to one side when the device is pulled in one direction and steer to the other side when the device is pulled in the opposite direction.

In FIG. 51D, device 260 has moved toward the opposite lateral side of the intervertebral foramen IF (hollow-tipped arrow) to remove additional ligamentum flavum LF tissue. In some embodiments, any or all blades 262, 262' of device 260 may have flat tops, which may help blades 262, 262' to slide or "skate" across the surface of bone as device 260 is reciprocated to cut through soft tissue. This sliding or skating motion may also help device 260 move from side to side within the intervertebral foramen IF.

In FIG. 51E, much of the ligamentum flavum LF has been removed, and blades 262, 262' are in a position to treat bone. In some cases, a physician may choose to continue using device 260 to remove bone, while in other cases a physician may wish to remove mostly or exclusively ligamentum flavum LF tissue. In various embodiments, the physician may determine when a desired amount of soft tissue and/or bone is removed by using tactile feedback from device 260, by removing device 260 to examine tissue trapped in device 260, by radiographic visualization such as fluoroscopy, by use of one or more sizing probes or other instruments to gauge the size of the intervertebral foramen IF, or any combination of such methods.

When a desired amount of tissue has been removed, device 260 may be removed from the patient to complete the procedure. As mentioned, in some embodiments, device 260 may be used to remove only ligamentum flavum LF tissue and then removed from the patient to end the procedure. In alternative embodiments, device 260 (or a differently configured device) may be used to remove both soft tissue and bone. In yet another alternative embodiment, a first device (for example, device 260) may be used to remove ligamentum flavum LF tissue, the first device may be removed from the patient, and a second device may be inserted and used to remove bone. Thus, in some embodiments, two different devices may be used in one procedure, with one device optimized for soft tissue removal and another device optimized for bone removal.

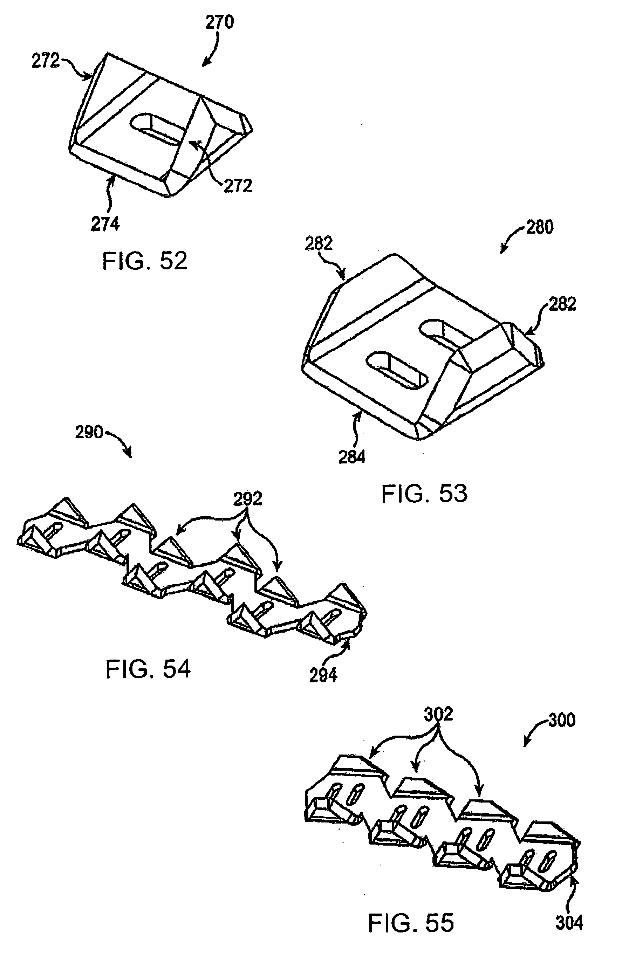

With reference now to FIGS. 52-55, various embodiments of blade structures are shown. For example, in an embodiment as in FIG. 52, a blade structure 270 may include two blades 272 extending substantially vertically from a base 274. In some embodiments, each set of two blades 272 and their associated base 274 may be made from one piece of material, with each blade 272 bending upward from base 274. Base 274 may provide a surface for attaching blades 272 to one side of a tissue modification device, such as my welding, attaching via adhesive and/or the like. In one embodiment, blades 272 may have beveled cutting edges and pointed tips, as shown, although any of a number of other blade configurations may alternatively be used.

In an alternative embodiment, as in FIG. 53, a blade structure 280 may again include two blades 282 extending substantially vertically from a base 284. In this embodiment, blades 282 have beveled edges and a flat, beveled top.

In another alternative embodiment, as in FIG. 54, a blade structure 290 may include any number of blades 292 coupled with a base 294. In this embodiment, twelve blades 292 are coupled with base 294, and base 294 has a back-and-forth (or "zig-zag") configuration.

In another alternative embodiment, as in FIG. 55, a blade structure 300 may include eight, flat-top blades 302 (or any other suitable number) coupled with a base 304 having a diagonal configuration. When base 304 is attached to a surface of a tissue modification device, blades 302 may be angled and/or laterally offset due to the diagonal configuration of base 304.

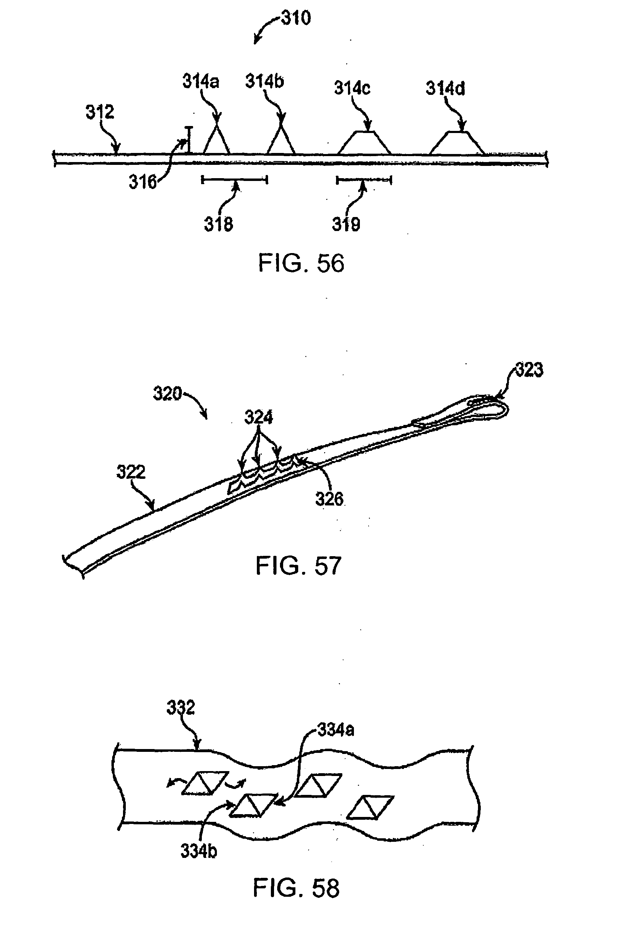

Referring now to FIG. 56, one embodiment of a tissue modification device 310 may include an elongate body flexible portion 312 and multiple blades 314 attached to one side of flexible portion 312 such that each blade 314 has a height 316 and a length 319, and such that a distance between two blades 314 defines a pitch 318. As mentioned previously, in various embodiments, blades 314 may have any of a number of shapes, such as pointed-tip 314a, 314b and flat-top 314c, 314d. Each blade 314 may also have a height 316, which may be defined as a distance between of first end of the blade 314, which is coupled with a first surface of flexible portion 312, and a second, cantilevered end of the blade 314. In some embodiments, for example, blades 314 each have a height ranging from about 0.5 mm to about 2.0 mm. In some embodiments, two or more blades may have different heights relative to one another. In one embodiment, for example, one or more sets of blades 314 may have a height optimized for addressing bone and one or more other sets of blades 314 may have a height optimized for addressing soft tissue. In one embodiment, shorter blades 314 may be positioned more distally on flexible portion 312, relative to higher blades 314 positioned more proximally. This placement of blades 314 may facilitate entry of device 310 into a tight anatomical location on a patient or around a tight corner.

Length 319 of each blade 314 may be defined as a distance between two blade edges. In various embodiments, blades 314 may have any suitable lengths, and a variety of blade lengths may be used in the same embodiment. Blades 314 may also have a pitch 318, defined as a distance from the beginning of an edge of one blade 314a to the beginning of an edge of a next adjacent blade 314b along device 310. In some embodiments, for example, pitch 318 may range from about 0.5 mm to about 4.0 mm. In various embodiments, any suitable combination of blade shapes, heights 316, lengths 319 and pitches 318 may be used.

With reference now to FIG. 57, in another embodiment, a tissue modification device 320 may include multiple blades 324 formed directly out of a flexible portion 322, thus creating an opening 326 in flexible portion 322. For example, blades 324 may be cut and bent out of flexible portion 322. Flexible portion 322 may also include a guidewire coupler 323. In this embodiment, flexible portion 322, blades 324 and guidewire coupler 232 are formed from one piece of material.

Referring to FIG. 58, in another alternative embodiment, multiple substantially vertical, substantially in-line blades 334 may be formed in a flexible portion 332 of a tissue modification device by cutting multiple flaps in flexible portion 332 and pulling them up to form blades 334 (curved, hollow-tipped arrows). In some embodiments, flexible portion 332 may be curved.

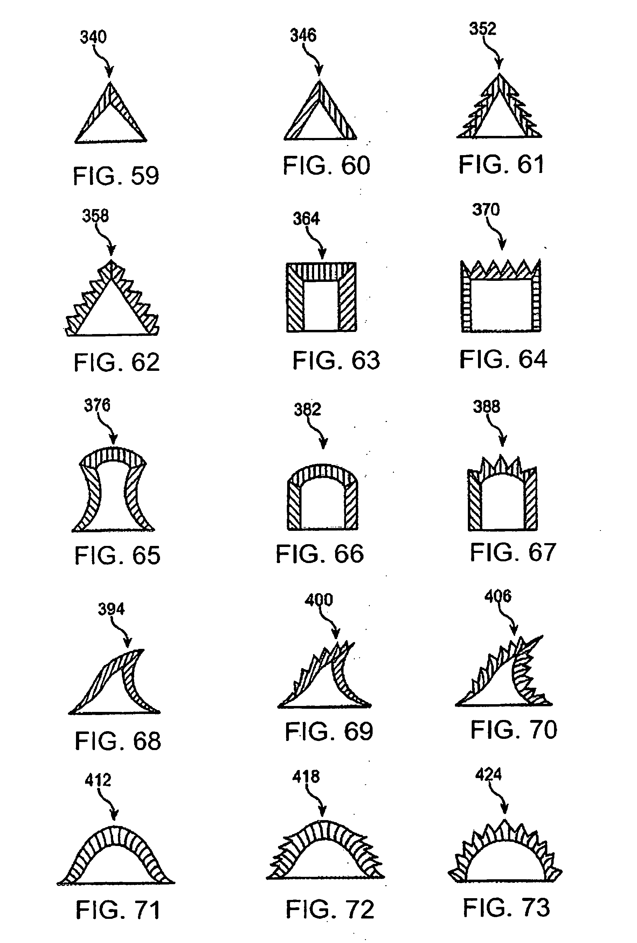

Referring now to FIGS. 59-76, a number of different embodiments of blades, which may be included in various embodiments of tissue modification devices, are shown. This is not meant to be an all-inclusive list, but instead is provided for exemplary purposes. Thus, other blades shapes and configurations not shown in FIGS. 59-76 may also be used in various embodiments of tissue modification devices.

The blade embodiments shown and described below generally have more than one cutting edge, and generally each edge of each blade is a cutting edge. In various alternative embodiments, however, a blade may have multiple edges, but not all the edges need be cutting edges. For example, in some embodiments a blade may have a cutting edge on one side and a dull edge on an opposite side, thus acting as a one-direction cutting blade. In another embodiment, a blade may have a front edge, a back edge and a top edge, and only the front and back edges might be cutting edges, with the top edge being dull, for example to facilitate the blade's riding along a bone surface. Generally, any edge of a blade described below may be, in alternative embodiments, a cutting edge or a non-cutting edge. Cutting edges, generally, may have any of a number of different configurations, such as beveled, pointed, serrated, saw-toothed and the like. Non-cutting edges may also have any of a number of different configurations, such as squared, rounded, notched or the like.

The blades of FIGS. 59-62 are all generally triangle-shaped. FIG. 59 shows a triangle-shaped, pointed-tip blade 340 with tapered cutting edges. FIG. 60 shows a triangle-shaped, pointed-tip blade 346 with straight cutting edges. FIG. 61 shows a triangle-shaped, pointed-tip blade 352 with downward-facing barbs on two cutting edges. FIG. 62 shows a triangle-shaped, pointed-tip blade 358 with saw-tooth cutting edges.

FIGS. 63 and 64 show square-shaped blades. FIG. 63 shows a square-shaped blade 364 with a flat-top cutting edge and straight vertical cutting edges. FIG. 64 shows a square-shaped blade 370 with straight vertical cutting edges and a crown-shaped (or serrated or saw-tooth) upper horizontal cutting edge.

The blades in FIGS. 65-67 all have convex-shaped upper cutting edges. In FIG. 65, blade 376 has a convex upper cutting edge and concave lateral cutting edges. In FIG. 66, blade 382 has a convex upper cutting edge and straight lateral (or vertical) cutting edges. In FIG. 67, blade 388 has a convex, crown-shaped (or serrated or saw-tooth) upper cutting edge and straight lateral cutting edges.

The blades in FIGS. 68-70 are all wave-shaped. The blade 394 of FIG. 68 has a wave shape and two smooth cutting edges. The blade 400 of FIG. 69 has a wave shape, one smooth cutting edge and one saw-tooth (or serrated) cutting edge. The blade 406 of FIG. 70 has a wave shape and two saw-tooth cutting edges.

FIGS. 71-73 all show rounded blades. In FIG. 71, blade 412 is rounded with a smooth cutting edge. In FIG. 72, blade 418 is rounded with downward facing barbs along a portion of its cutting edges. In FIG. 73, blade 424 is rounded with a saw-tooth (or serrated) cutting edge.

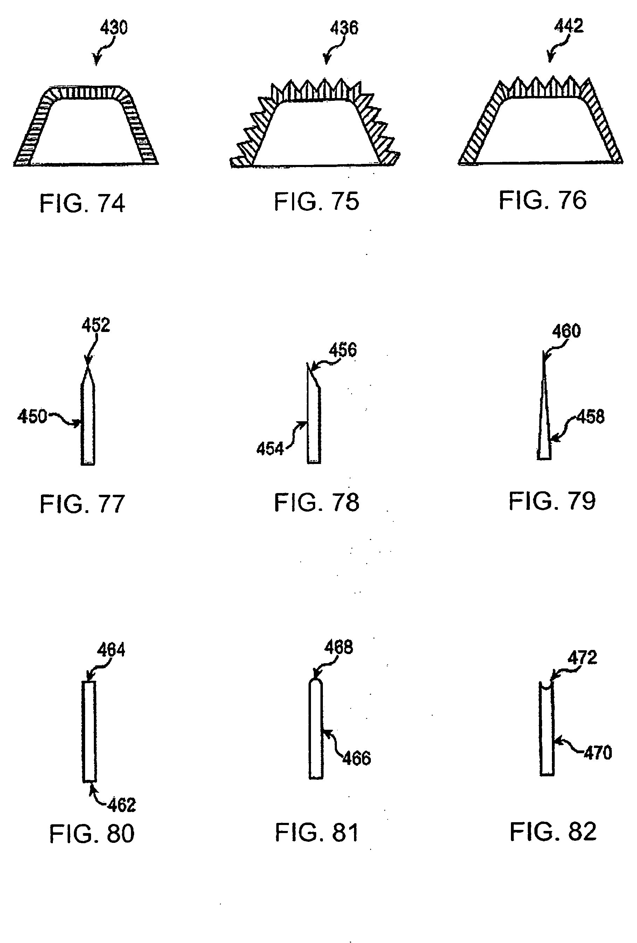

The blades of FIGS. 74-76 are all trapezoidal in shape. In FIG. 74, blade 430 has a trapezoidal shape and straight/smooth cutting edges. In FIG. 75, blade 436 has a trapezoidal shape and saw-tooth (or serrated) cutting edges. In FIG. 76, blade 442 has a trapezoidal shape and straight lateral cutting edges with a saw-tooth (or serrated) upper cutting edge. Again, the foregoing examples are provided for exemplary purposes, and in various embodiments, tissue modification devices may include any alternative blade shapes and configurations.

FIGS. 77-82 are cross-sectional views of a number of different blade embodiments, looking from an end-on perspective. According to various embodiments, blades may have any of a number of different upper cutting surfaces, and FIGS. 77-82 illustrate several examples of such surfaces. In FIG. 77, for example, blade 450 includes an upper cutting edge having a double-bevel configuration. The blade 454 in FIG. 78 has a single-bevel upper cutting edge 456. In FIG. 79, blade 458 has a tapered shape that ends in upper cutting edge 460.

In some embodiments, a blade may have an upper surface that is not sharp or pointed. Such an upper surface may help such a blade to slide or skate off of a bony surface, thus facilitating steering of a tissue modification device. For example, in FIG. 80, blade 462 has a flat upper surface 464. In FIG. 81, blade 466 has a rounded (or convex) upper surface 468. In FIG. 82, blade 470 has a concave upper surface 472. Again, any other suitable blade shape may be used in various alternative embodiments.

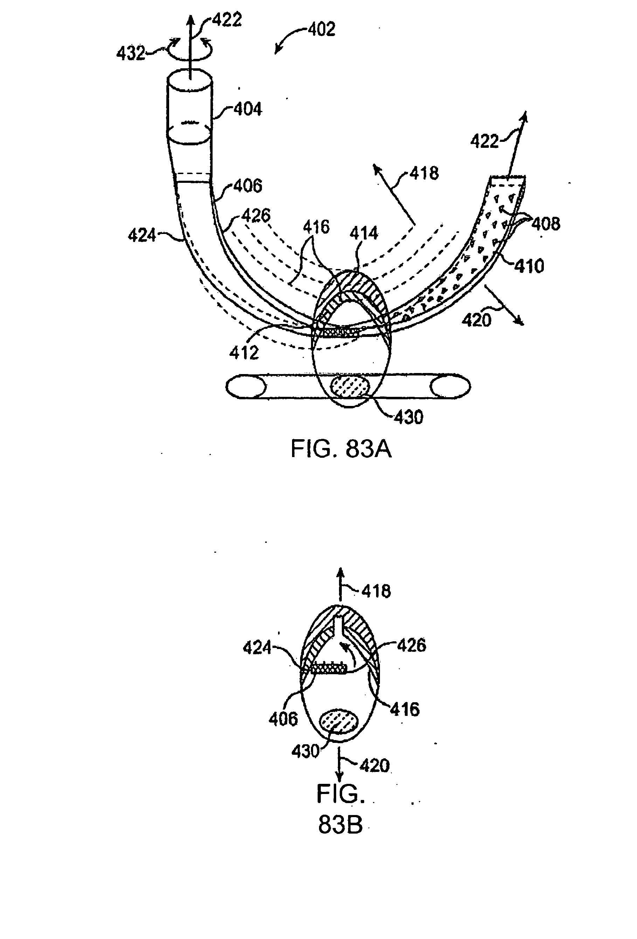

Referring now to FIGS. 83A and 83B, a tissue modification device 402 has a rigid proximal shaft portion 404 from which a flexible portion 406 extends axially. A plurality of tissue modification elements in the form of blades 408 extend from a first surface 410 of flexible portion 406, as described above. Flexible portion 406 is advanced into a patient body so that first surface 410 is bent over a target tissue, with the target tissue here comprising both ligament 412 and bone 414. First surface 410 of flexible portion 406 is wrapped over an at least partially convex surface 416, with the convexity of the surface defining an inward orientation 418 and an outward orientation 420. Hence, axial tension 422 on the flexible portion 406 causes the first surface 410 to move inwardly toward the target tissue 412, 414.

Referring still to FIGS. 83A and 83B, the surface 416 of the target tissue need not, and often will not, be substantially cylindrical, but will often instead have portions that are more inward 418, and other portions that are more outward 420. For example, a first portion or region of the surface 416 adjacent a first edge 424 of flexible portion 406 may be significantly more outward 420 than a region of the surface that is adjacent an opposed edge 426 and engagement between the flexible portion and tissue surface. As a result of the axial tension 422 in the flexible portion 406, this difference can cause the flexible portion to rotate about its central axis. Continued reciprocation of the flexible portion when its rotational orientation is not adequately controlled could cause an edge 426 of the flexible portion to cut laterally into target tissues as illustrated in FIG. 83B, or even inadvertent flipping of the flexible portion which might expose non-target tissue 430 to damage from the cutting blades along first surface 410, rather than effecting controlled volumetric removal of the target tissue.

To inhibit uncontrolled rotation of the flexible portion 406, the rigid shaft of proximal portion 404 significantly improves the control over both the orientation and position of the flexible portion, in part by transmitting torque 432 from the proximal handle to the treatment site within the patient. By rotating (or restraining) the proximal handle about the axis of the shaft, torque is transmitted down the shaft and to the flexible portion adjacent the target tissue. The torque can be transmitted so as to inhibit rolling or flipping of the flexible portion, and can also be used to intentionally alter an orientation of the flexible portion and tissue modifying members. The proximal handle and/or proximal portion may have an asymmetric shape or some asymmetric indicia that identifies the orientation of the tissue modifying members to enhance the physician's control over the orientation of tissue being modified and/or removed.

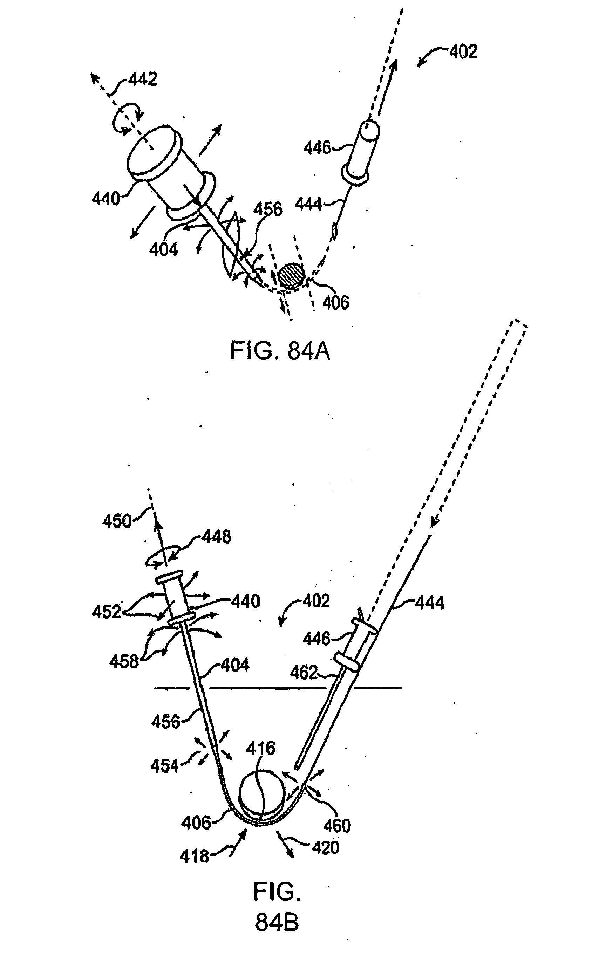

Referring now to FIGS. 84A and 84B, additional aspects of the structure and use of rigid shaft proximal portion 404 to control the location and orientation of distal flexible portion 406 can be understood. As generally described above, tissue modification tool 402 is generally positioned for use with rigid portion 404 extending a proximal handle 440 through an open or minimally invasive surgical axis site to flexible portion 406, with the flexible portion often extending distally from an axis 442 of the proximal portion. The distal flexible portion 406 also has a central axis which extends around a target tissue to a distal end that is coupled to a guidewire 444 extending out of the patient, with a distal handle 446 being axially affixable to the guidewire so that tension can be applied to the flexible portion 406 by pulling upward on the proximal and distal handles 440, 446.

As described above, torqueing the shaft of rigid portion 404 about its axis using handle 440 (as schematically illustrated by arrows 448) can help to orient the tissue treatment member(s) along the first surface 410 of flexible portion 406 toward a target region of the target tissue. Additionally, it will often be desirable to shift flexible portion 406 laterally relative to its central axis, that is, into and/or out of the illustration of FIG. 84B. Handle 440 can be used to help move flexible portion 406 using one or both of two techniques. First, handle 440 can be pushed laterally relative to the axis 450 of the rigid proximal shaft portion 404 as illustrated by arrows 452. Where handle 440 laterally translates the shaft without rotating of the shaft, end 454 of rigid portion 404 may also translate laterally, thereby laterally shifting the flexible portion 406. Alternatively, handle 440 may be used to pivot the rigid portion 404 about an effective pivot point 456 (as schematically illustrated by curving arrows 458), similarly effecting lateral movement of the end 454 of the rigid portion within the patient. Some combination of lateral movement of the overall rigid portion 404 will often be combined with some pivoting of the rigid portion. The pivot point 456 is not necessarily at a fixed location in space, and may move somewhat as the tissues adjacent the tissue modification tool 402 are displaced and/or compressed.

As described above, guidewire 444 advantageously allows tension to be applied to a distal end 460 of flexible portion 406, optionally allowing the flexible portion to be shifted and/or positioned along its curving access for treatment of a target tissue, as well as allowing distraction of target tissues, reciprocation of the tissue modification elements and flexible portion against a target tissue, and the like. To enhance lateral and rotational control over the flexible portion 406, and particularly the length of the flexible portion close to its distal end 460, a second rigid shaft 462 may be affixed to distal handle 446. The second shaft 462 may have a central lumen that receives guidewire 444 therethrough. Second shaft 462 may then be manipulated as described above regarding the rigid portion 404, allowing the distal end 460 of the flexible portion to be shifted in coordination with the shifting effected by the rigid portion 404. This may enhance overall control over the lateral movement of flexible portion, optionally using the pivoting and/or lateral movement techniques described above. The second rigid shaft 462 will often have a distal end with a profile suitable for advancing distally over guidewire 44 toward the target tissue, and may also torquably engage the distal end of flexible portion 406 so as to allow the distal end to be torqued about the longitudinal axis of the flexible portion and guidewire (such as by providing a slot in the inserted end of second shaft 462 to torquably receive the distal end of the flexible portion).

Referring again to FIGS. 51A-51E, it will often be desirable to remove target tissue from a tissue region 259 which is wider than an adjacent tissue modification device 260. Additionally, it may be desirable to reorient the tissue modification members carried by a flexible portion of a tissue modification device 260 so as to treat portions of the target tissue that are at different angles. As described above, tensioning of tissue modification device 260 using the proximal and distal handles can urge the tissue modifying members toward a first region of the target tissue, such as the region being engaged by blades 262, 262' in FIG. 51B. As this tissue is removed, the tension will tend to keep the tissue modification device 260 at the removed tissue location. Optionally, the orientation of the tissue modification device 260 may be rotated about a central axis of the flexible portion of the tissue modification device by rotation of rigid portion 404 (see FIGS. 83A, 84A), resulting in lateral rotation of the flexible portion and tissue modification elements carried thereby in a counter-clockwise direction (see FIG. 51C) wherein a clockwise direction (see FIG. 51D). Additionally, lateral translation and/or pivoting of the rigid portion 404 about pivot point 456 may be used to laterally shift or translate the tissue treatment device 260.

Lateral shifting of the flexible portion may be facilitated (for example) by including tissue modification devices or blades having sufficient length to extend through ligament target tissue such as the ligamentum flavum, and by including tips on at least some of the tissue modification devices or blades that are large enough to avoid penetrating into underlying bone. This may allow the flexible substrate to ride over the tough ligament, facilitating lateral movement of the outermost blades into target ligament tissues. Lateral shifting of the flexible portion may also be facilitated by a flexible substrate structure which is relatively stiff in one lateral orientation (specifically, along the major surfaces) and more flexible in another lateral orientation (transverse to the major surfaces, so as to allow the flexible member to bend over the target tissue with a major surface oriented toward the target tissue). Advantageously, such selective lateral flexibility and lateral stiffness can be readily provided by a thin, flat substrate having a cross-section that includes a much larger moment in one orientation (for example, bending in the plane of the major surfaces) than another (for example, bending in the plane of the smaller edges).

Although various illustrative embodiments are described above, any of a number of changes may be made to various embodiments without departing from the scope of the invention as described by the claims. Optional features of various device and system embodiments may be included in some embodiments and not in others. Therefore, the foregoing description is provided primarily for exemplary purposes and should not be interpreted to limit the scope of the invention as it is set forth in the claims.

## Claims

1. A flexible tissue-modification device (10, 160) for removing target tissue from around a curved pathway within a patient by manually reciprocating both ends of the device while urging it against the target tissue, the device comprising:
a flexible elongate body (14, 162) having an axial length, a width and a thickness, wherein the axial length is greater than the width, and the width is greater than the thickness;
**characterised in that** the body has two parallel sides formed of wire (4407) extending in the axial direction; and **in that**
a plurality of cutting surfaces (4403) is connected to and extending between the two sides formed of wire across the width of the device.

2. The device of claim 1, further comprising a lower connecting surface and an upper connecting surface, each of the upper and lower connecting surfaces extending between the two sides and comprising a mesh (4409).

3. The device of claim 1, further comprising a mesh (4409) attached to the sides formed of wire to form a tissue collection region (4311).

4. The device of claim 1, wherein the sides formed of wire comprise two parallel wires.

5. The device of claim 1, wherein the elongate body is ribbon-shape, having a width that is greater than the thickness.

6. The device of claim 1, further comprising a guidewire coupler (18, 168) at the distal end of the device.

7. The device of claim 1, further comprising a handle (20, 170) or a handle attachment region at the proximal end of the device.

8. The device of claim 1, further comprising at least one protective side guard extending along the length of the flexible elongate body.

9. The device of claim 1, further comprising an opening (4405) between adjacent cutting surfaces (4403).

10. The device of claim 1, wherein each cutting surface (4403) comprises one or more cutting edge (3801) that projects from the cutting surface.

11. The device of claim 10, wherein the cutting edges (3801) on adjacent cutting surfaces are axially offset from each other.

12. The device of claim 1, further comprising a tissue collection region (4311).

## Patentansprüche

1. Flexible Gewebemodifikationsvorrichtung (10, 160) zur Entfernung von Zielgewebe aus einem Umgebungsbereich eines gekrümmten Pfades in einem Patienten durch manuelles Hin- und Herbewegen beider Enden der Vorrichtung, während sie gegen das Zielgewebe gedrückt wird, wobei die Vorrichtung aufweist:
einen flexiblen länglichen Körper (14, 162) mit einer axialen Länge, einer Breite und einer Dicke, wobei die axiale Länge größer als die Breite ist, und die Breite größer als die Dicke ist;
**dadurch gekennzeichnet, dass** der Körper zwei parallele Seiten hat, die aus sich in der axialen Richtung erstreckendem Draht (4407) ausgebildet sind; und **dadurch** dass
mehrere Schneidflächen (4403) mit den zwei aus Draht ausgebildeten Seiten verbunden sind und sich dazwischen über die Breite der Vorrichtung erstrecken.

2. Vorrichtung nach Anspruch 1, welche ferner eine untere Verbindungsfläche und eine obere Verbindungsfläche aufweist, wobei sich jede von der oberen und unteren Verbindungsoberflächen zwischen den zwei Seiten erstreckt, und ein Sieb (4409) aufweist.

3. Vorrichtung nach Anspruch 1, die ferner ein an den aus Draht geformten Seiten befestigtes Sieb aufweist, um einen Gewebesammelbereich (4311) auszubilden.

4. Vorrichtung nach Anspruch 1, wobei die aus Draht geformten Seiten zwei parallele Drähte ausweisen.

5. Vorrichtung nach Anspruch 1, wobei der längliche Körper eine Bandform mit einer Breite hat, die größer als die Dicke ist.

6. Vorrichtung nach Anspruch 1, die ferner eine Führungsdrahtkupplung (18, 168) an dem distalen Ende der Vorrichtung aufweist.

7. Vorrichtung nach Anspruch 1, die ferner einen Griff (20, 170) oder einen Griffbefestigungsbereich an dem proximalen Ende der Vorrichtung aufweist.

8. Vorrichtung nach Anspruch 1, die ferner wenigstens eine schützende Seitenabdeckung aufweist, die sich entlang der Länge des flexiblen länglichen Körpers erstreckt.

9. Vorrichtung nach Anspruch 1, die ferner eine Öffnung (4405) zwischen benachbarten Schneidflächen (4403) aufweist.

10. Vorrichtung nach Anspruch 1, wobei jede Schneidfläche (4403) einen oder mehrere Schneidränder (3801) aufweist, die aus der Schneidfläche hervorstehen.

11. Vorrichtung nach Anspruch 10, wobei die Schneidränder (3801) auf benachbarten Schneidflächen axial zueinander versetzt sind.

12. Vorrichtung nach Anspruch 1, die ferner einen Gewebesammelbereich (4311) aufweist.

## Revendications

1. Dispositif de modification de tissu flexible (10, 160) destiné à retirer un tissu cible du pourtour d'une voie courbe chez un patient par un mouvement de va-et-vient manuel des deux extrémités du dispositif tout en le poussant contre le tissu cible, le dispositif comprenant :
un corps allongé flexible (14, 162) ayant une longueur axiale, une largeur et une épaisseur, la longueur axiale étant plus grande que la largeur, et la largeur étant plus grande que l'épaisseur ;
**caractérisé en ce que** le corps a deux côtés parallèles formés d'un fil (4407) s'étendant dans la direction axiale, et **en ce qu'**une pluralité de surfaces de coupe (4403) est raccordée et s'étend entre les deux côtés formés de fil sur la largeur du dispositif.

2. Dispositif selon la revendication 1, comprenant en outre une surface de raccordement inférieure et une surface de raccordement supérieure, chacune des surfaces de raccordement supérieure et inférieure s'étendant entre les deux côtés et comprenant un maillage (4409).

3. Dispositif selon la revendication 1, comprenant en outre un maillage (4409) fixé aux côtés formés de fil pour former une région de recueil de tissu (4311).

4. Dispositif selon la revendication 1, dans lequel les côtés formés de fil comprennent deux fils parallèles.

5. Dispositif selon la revendication 1, dans lequel le corps allongé est de type ruban, ayant une largeur qui est supérieure à l'épaisseur.

6. Dispositif selon la revendication 1, comprenant en outre un coupleur de fil guide (18, 168) sur l'extrémité distale du dispositif.

7. Dispositif selon la revendication 1, comprenant en outre une poignée (20, 170) ou une région de fixation de poignée à l'extrémité proximale du dispositif.

8. Dispositif selon la revendication 1, comprenant en outre au moins un protecteur latéral s'étendant sur la longueur du corps allongé flexible.

9. Dispositif selon la revendication 1, comprenant en outre une ouverture (4405) entre les surfaces de coupes adjacentes (4403).

10. Dispositif selon la revendication 1, dans lequel chaque surface de coupe (4403) comprend un ou plusieurs bords coupants (3801) qui se projettent de la surface de coupe.

11. Dispositif selon la revendication 10, dans lequel les bords de coupe (3801) sur des surfaces de coupe adjacentes sont décalés axialement les uns des autres.

12. Dispositif selon la revendication 1, comprenant en outre une région de recueil de tissu (4311).
